# EUROPEAN PATENT APPLICATION

(11) **EP 2 592 083 A1**
(43) Date of publication of application: **15.05.2013**
(21) Application number: 11782976.2
(22) Date of filing: 20.05.2011
(51) Int. Cl.: C07D 487/04, A61K 31/519, A61P 35/00

(54) **FUSED QUINAZOLINE DERIVATIVES AND USES THEREOF**

(30) Priority: 21.05.2010 CN 201010182895
(71) Applicant: Shenzhen Salubris Pharmaceuticals Co., Ltd., Shenzhen, Guangdong 518040 (CN); Shanghai Institute of Pharmaceutical Industry, Shanghai 200040 (CN)
(72) Inventor: LI, Jianqi, Shanghai 200040 (CN); ZHANG, Zixue, Shanghai 200040 (CN); XIE, Peng, Shanghai 200040 (CN); ZHANG, Qingwei, Shanghai 200040 (CN)
(74) Representative: Crowhurst, Charlotte Waveney
(86) International application number: PCT/CN2011/074434
(87) International publication number: WO 2011/144059

(57) **Abstract**

Fused quinazoline derivatives and uses thereof as protein tyrosine kinase inhibitors and aurora kinase inhibitors are disclosed. Said protein tyrosine kinase inhibitors and aurora kinase inhibitors can be used in treating cancers, leukaemia and the diseases relevant to differentiation and proliferation. Said protein tyrosine kinase and aurora kinase dual inhibitors are the compounds represented by the following general formula or salts thereof.

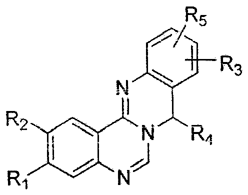

## Description

This application claims the benefit of priority Chinese Application No. CN 201010182895.7, filed on May 21, 2010, the disclosure of which is hereby incorporated by reference in its entirety.

### Field

This disclosure relates to a series of active pharmaceutical ingredients chosen from polycyclic quinazolines disclosed herein, pharmaceutically acceptable salts thereof, and hydrates of the pharmaceutically acceptable salts, and the preparation and use thereof.

### Background of the invention

The protein tyrosine kinase family, either as transmembrane receptors or as cytoplasmic proteins, is widely involved in cell signaling transduction through phosphorylation and dephosphorylation processes. In the process of carcinogenesis, mutation or over expression of protein tyrosine kinases can transform normal cells into cancer cells, and promote tumor cell mitosis and growth. Therefore, inhibitors of these enzymes can be used to treat cancer. It is known that the epidermal growth factor receptor (EGFR) tyrosine kinase family includes 4 main members, ErbB-1, ErbB-2, ErbB-3, and ErbB-4. At least one of ErbB receptors are over-expressed in about 60% of known tumors, especially in solid tumors such as lung cancer, breast cancer, pancreatic cancer, prostate cancer, stomach cancer, ovarian cancer, etc. It is also known that the vascular endothelial growth factor receptor (VEGFR) family, which includes five main members, three of which (VEGFR-1, VEGFR-2, and VEGFR-3) belong to the tyrosine kinase receptor superfamily. High expression of VEGFR and angiogenesis are prerequisite conditions for tumor growth. Thus, blocking the signal transduction of VEGFR and inhibiting angiogenesis is a potentially effective method for cancer therapy. Therefore, EGFR and VEGFR tyrosine kinase inhibitors can be used for the treatment of cancers in human.

Aurora kinases are important serine/threonine kinases in the cell mitotic regulatory network. The aurora kinase family can be categorized as Aurora A, Aurora B, and Aurora C. They play an important role in regulating mitosis. Abnormal expression of aurora kinases can interfere with mitosis, which may lead to genetic instability and tumor growth. A series of aurora kinase inhibitors such as Hesperadin, ZM447439, MK0457 (also known as VX-680), AZD1152, MLN8054, PHA-739358, show good inhibitory activity on Aurora A and Aurora B, and have been used in preclinical or phase I clinical trials.

US5747498 reported a series of quinazolines derivatives as EGFR inhibitors, which are as follow: 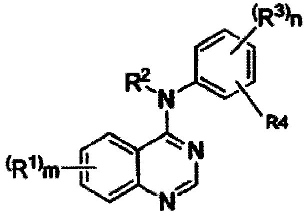 wherein, m=1, 2, 3. R¹ is independently chosen from hydrogen, halogen, hydroxy, amino, hydroxyamino, carboxyl, C₁-C₄ alkoxy carbonyl, nitro, guanidino, ureido, carbamoyl, cyano, trifluoromethyl, group denoted as C₁₋₄ alkenyl-W-Phenyl-alkyl, wherein W is chosen from single bond, O, S, NH; R⁴ is chosen from azide group, substituted or unsubstituted ethynyl.

WO9749688 disclosed a series of tricyclic derivatives as EGFR inhibitors, which are as follow: 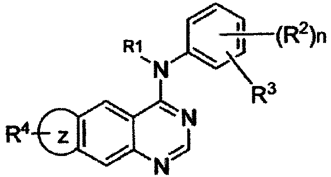 wherein, n=0, 1, 2; R¹ is hydrogen or C₁-C₆ alkyl optionally substituted by 1 to 3 substituents independently selected from the group consisting of halogen, C₁-C₄ alkoxy, -NR⁶R⁷, and -SO₂R⁵; each R² is independently selected from the group consisting of halogen, hydroxy, C₁-C₆ alkoxy, - NR⁶R⁷, and C₁-C₄ alkoxy, wherein said alkyl group and the alkyl moieties of said R² groups are optionally substituted by 1 to 3 substituents independently selected from the group consisting of halogen, C₁-C₄ alkoxy, -NR⁶R⁷, and -SO₂R⁵; R³ is azido or -(ethynyl)-R⁸ wherein R⁸ is hydrogen or C₁-C₆ alkyl optionally substituted by hydroxy, -OR⁶, or -NR⁶R⁷; R⁴ is H, C₁-C₄ alkyl, (C₁-C₄ alkoxy)C₁-C₄ alkyl, C₁-C₄ alkanoyl, C₁-C₄ alkoxy or -S(O)x(C₁-C₄ alkyl) wherein x is 1 to 2, and wherein said alkyl group and the alkyl moieties of said R⁴ groups are optionally substituted by 1 to 3 halogens; each R⁵ is C₁-C₄ alkyl optionally substituted by 1 to 3 halogens; R⁶ and R⁷ are independently selected from hydrogen and R⁵; and, Z is a 5 to 8 membered fused ring that includes 0 to 3 heteroatoms selected from O, S and N.

WO2007083096 disclosed a series of quinazolines derivatives as aurora kinases inhibitors, which are shown bellow: 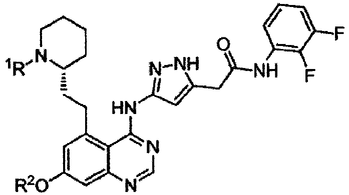 wherein, R¹ is hydrogen or methyl, R² is methyl or ethyl.

Besides, many compounds also show EGFR or aurora kinases inhibitory activity, hitherto, many quinazoline compounds reported as EGFR inhibitors or aurora kinases inhibitors show certain anti-cancer activity, three among them have been marketed and gained huge successes, they are gefitinib, erlotinib hydrochloride and lapatinib di-p-tosylate, meanwhile, several aurora kinases inhibitors potential are being used in phase I or II trials.

Nine small molecule tyrosine kinase inhibitors are currently marketed as anticancer drugs. The structures of those nine tyrosine kinase inhibitors are shown below:

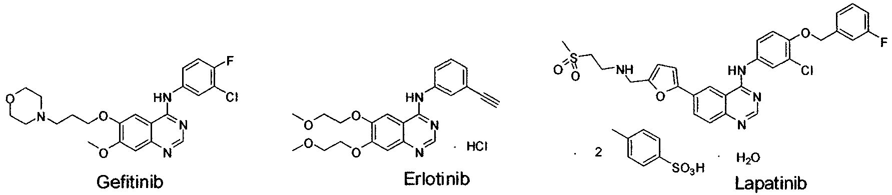

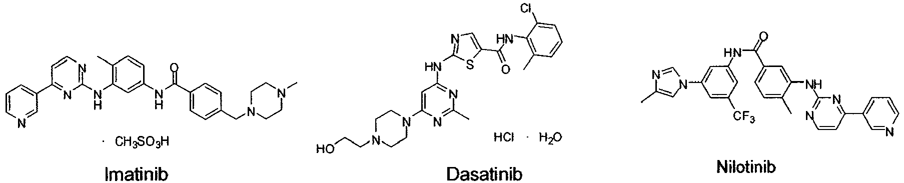

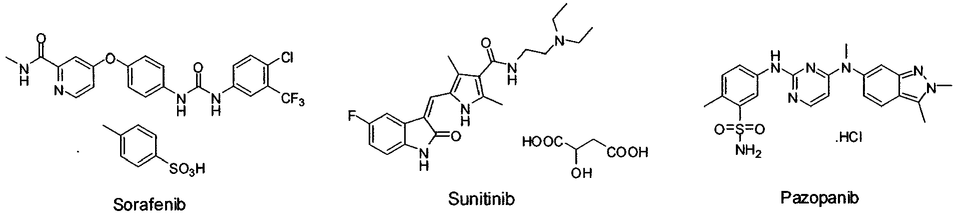

Although the anti-cancer compounds above have contributed a great deal to the field, research continues in this area to improve anti-cancer drugs.

### Detailed description of the invention

This invention involves an active pharmaceutical ingredient chosen from polycyclic quinazolines, pharmaceutical acceptable salts thereof, or hydrates of the pharmaceutically acceptable salts, which show certain tyrosine kinase and aurora kinases inhibitory activities.

The disclousure disclouses an active pharmaceutical ingredient chosen from polycyclic quinazolines of formula V 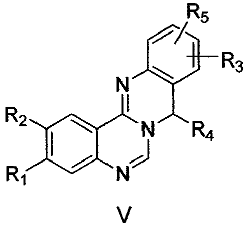 wherein, R₁ or R₂ is independently chosen from hydrogen, halogen, amino, nitro, hydroxamino, carboxyl, C₁-C₄ alkoxy carbonyl, C₁-C₄ alkyl, C₁-C₄ acylamino , (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₃ or R₅ is independently chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted benzyloxy, substituted heterocyclic;
R₄ is chosen from hydrogen, trifluoromethyl, C₁-C₄ alkyl and C₁-C₄ alkoxy;
R₁, R₂, R₃, R₄ and R₅ are not simultaneously hydrogen.

In one embodiment, R₁ is chosen from hydrogen, halogen, amino, nitro, hydroxamino, carboxyl, C₁-C₄ alkoxy carbonyl, C₁-C₄ alkyl, C₁-C₄ acylamino , (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₂ is chosen from hydrogen, halogen, amino, nitro, hydroxamino, carboxyl, C₁-C₄ alkoxy carbonyl, C₁-C₄ alkyl, C₁-C₄ acylamino , (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₃ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted benzyloxy, substituted heterocyclic;
R₄ is chosen from hydrogen, trifluoromethyl, C₁-C₄ alkyl and C₁-C₄ alkoxy;
R₅ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted benzyloxy , substituted heterocyclic;
R₁, R₂, R₃, R₄ and R₅ are not simultaneously hydrogen.

In one embodiment, the pharmaceutically acceptable salts are preferably chosen from hydrochloride, hydrobromide, sulfate, acetate, lactate, tartrate, tannate, citrate, triflouroacetate, malate, maleate, succinate, p-toluenesulfonate, and mesylate.

In one embodiment, the hydrates of the pharmaceutically acceptable salts preferably have a hydrate number range from 0.5 to 3.0.

The term "halogen" as disclosed herein refers to fluorine, chlorine, bromine, and iodine.

The term "amino" is an -NH₂. The term "substituted amino" is a group which one or two hydrogen atoms of -NH₂ are independently substituted. For example, one or two hydrogen atoms of -NH₂ are independently substituted by alkyl, alkenyl, alkynyl, or alkoxy, etc. For another example,the term C₁-C₄ substituted amino" as disclosed herein refers to one or two hydrogen atoms of -NH₂ are independently substituted by C₁-C₄ alkyl, C₂-C₄ alkenyl, C₂-C₄ alkynyl, or C₁-C₄ alkoxy.In one embodiment, the C₁-C₄ alkyl amino is preferably chosen from methylamino, ethylamino, n-propylamino, iso-propylamino, n-butylamino, iso-butylamino, sec-butylamino.

The term "alkoxy" as disclosed herein refers to alkyl ether radical. In one embodiment, the alkoxy is preferably chosen from C₁-C₄ alkoxy, substituted and unsubstituted benzyloxy, pyrrolidine-1-yl-(C₂-C₄)alkoxy, morpholino-1-yl-(C₂-C₄)alkoxy, piperazin-1-yl-(C₂-C₄)alkoxy, N-methyl piperazine-1-yl-(C₂-C₄) alkoxy and piperidine-1-yl-(C₂-C₄)alkoxy. Wherein, the C₁-C₄ alkoxy as described herein is preferably chosen from methoxyl, ethoxyl, n-propoxyl, iso-propoxyl, n-butoxyl, iso-butoxyl, methoxyethoxyl, ethoxymethoxyl; the C₂-C₄ alkoxy described herein can be chosen from ethoxyl, n-propoxyl, iso-propoxyl, n-butoxyl, iso-butoxyl, methoxyethoxyl, ethoxymethoxyl; the substituted benzyloxy as disclosed herein refers to a group where at least one hydrogen atom of benzene ring is independently substituted, for example, one, two, three or four hydrogen atoms of benzene ring can be substituted by halogen, hydroxyl, nitro, cyano, C₁-C₄ alkoxy, C₁-C₄ alkyl and amino, for a further example, the substituted benzyloxy is 3-F-benzyloxy.

In one embodiment, the term "C₁-C₄ alkoxy carbonyl" as disclosed herein is preferably chosen from ethoxy methylcarbonyl denoted as formula CH₃OC(O)-, ethoxy methylcarbonyl denoted as formula CH₃CH₂OC(O)-, n-propyl methyl carbonyl, iso-propyl methyl carbonyl, n-butyl methyl carbonyl.

The term "alkyl" as disclosed herein can be branched or non-branched chain alkyl or cyclic alkyl having a specified carbon atom number. For example, C₁-C₄ alkyl is preferably chosen from methyl, ethyl, n-propyl, iso-propyl, cyclopropyl, n-butyl, iso-butyl, sec-butyl, tert-butyl, cyclobutyl, methoxyethyl, methoxy n-propyl, ethoxymethyl, ethoxyethyl, n-propoxymethyl, iso-propoxymethyl.

The term "acyl amino" as disclosed herein refers to -C(O)NH-. For example, C₁-C₄ acyl amino is preferably chosen from acetamido denoted as formula CH₃C(O)NH-, n-propionamido denoted as formula CH₃CH₂C(O)NH-, n-butanacylamino , iso-butanacylamino .

The term "sulfonyl" as disclosed herein refers to -SO₂-; the term "C₁-C₄ sulfonyl" is a group which "C₁-C₄ alkyl" is connected to "sulfonyl." For example, the term C₁-C₄ sulfonyl is preferably chosen from methyl sulfonyl (abbreviated as "Ms"), ethyl sulfonyl, n-propyl sulfonyl, iso-propyl sulfonyl, n-butyl sulfonyl, iso-butyl sulfonyl, and sec-butyl sulfonyl.

The term "aryl" as disclosed herein refers to a carbocyclic aromatic system containing one, two, three or more rings, wherein, the rings may be substituted or un substituted, or attached together with a pendent manner, or may be fused. For example, an aryl may be chosen from phenyl, naphthyl, anthryl, phenanthryl, diphenyl, 3-fluorine phenyl, 2-bromine phenyl, 3-chlorine naphthyl, etc.

The term "aryl sulfonyl" as disclosed herein means that the "aryl" is connected to the "sulfonyl".

The term "substituted phenyl" as disclosed herein refers to a group where at least one hydrogen atom of benzene ring is independently substituted. For example, substituted phenyl is preferably chosen from phenyl substituted by one, two, three or four substituents, wherein the substituents can be independently chosen from halogen, hydroxyl, nitro, cyano, C₁-C₄ alkoxy, C₁-C₄ alkyl, and amino. For a further example, the substituted phenyl can be chosen from chosen from 3-fluorine phenyl, 2-bromine phenyl, etc.

The term "heterocyclic" as disclosed herein is an aliphatic ring containing one or more, for example one, two, three, and four, heteroatoms in the ring, wherein the heteroatom is independently nitrogen, oxygen, or sulfur. The rings may be saturated, or partially unsaturated. For example, the term "heterocyclic" can be a five-membered heterocyclic or a six-membered heterocyclic ring. The "heterocyclic" may be, for example, furan, pyrrolidine, dihydro-pyrazole, piperidine, piperazine, morpholino, imidazole, or pyridine. The "substituted heterocyclic" as disclosed herein is the hydrogen atoms of the heterocycle can be independently substituted by one or more, e.g., two, three, or four, substituents. For example, the substituted heterocyclic can be 5-((2-(methylsulfonyl)ethylamino)methyl)furyl as shown in the following structure:

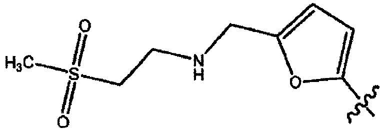

The term "unsaturated aliphatic chain" as disclosed herein refers to an unsaturated hydrocarbon having a specified number of carbon atoms. It can be a non-branched chain, branched chain, or cyclic unsaturated hydrocarbon chain. The "unsaturated aliphatic chain" can be, for example, C₁-C₄ unsaturated aliphatic chain chosen from vinyl, n-propenyl, iso-propenyl, n-butenyl, iso-butyleneyl, sec-butenyl, ethynyl, propinyl, n-butynyl or branched compounds therefor. For another example, the "unsaturated aliphatic chain" can be C₂-C₄ unsaturated aliphatic chain chosen from ethynyl, propynyl, n-butynyl, iso-butynyl.

The five-membered aliphatic ring as disclosed herein refers to a substituted or unsubstituted five-membered ring. The six-membered aliphatic ring as used herein refers to a substituted or unsubstituted six-membered ring.

The term "alkyl sulfonic ester" as disclosed herein is a group ester composed by alkyl alcohol and alkylsulfonic acid. In one embodiment, the alkyl alcohol is preferably chosen from C₁-C₄ alkyl alcohol, such as methanol, ethanol, n-propanol, iso-propanol, n-butanol, etc. In one embodiment, the alkylsulfonic acid is preferably chosen from C₁-C₄ alkylsulfonic acid, such as methylsulfonic acid, ethylsulfonic acid, etc. In one embodiment, the alkyl sulfonate is methanol methanesulfonic acid ester (denoted as -CH₂OMs).

Unless otherwise specified, a non-specified number for a given substituent as used herein means "one or more".

In one embodiment, R₁ is chosen from hydrogen, halogen, amino, nitro, hydroxamino, carboxyl, ethoxy methylcarbonyl, n-propyl, (E)-4-(dimethylamino)-2-butene acylamino, methoxyl, 2-methoxy-ethoxy, trifluoromethyl, 3-fluoro-benzyloxy, pyrrolidine-1-yl-2-propoxy, morpholino-1-yl-2-propoxy, piperazin-1-yl-2-propoxy, N-methyl piperazine-1-yl-2-propoxy, piperidine-1-yl-2-propoxy, 5-((2-(methylsulfonyl)ethylamino)methyl)furyl;
R₂ is chosen from hydrogen, halogen, amino, nitro, hydroxamino, carboxyl, ethoxy methylcarbonyl, n-propyl, (E)-4-(dimethylamino)-2-butene acylamino, methoxyl, 2-methoxy-ethoxy, trifluoromethyl, 3-fluoro-benzyloxy, pyrrolidine-1-yl-2-propoxy, morpholino-1-yl-2-propoxy, piperazin-1-yl-2-propoxy, N-methyl piperazine-1-yl-2-propoxy, piperidine-1-yl-2-propoxy, 5-((2-(methylsulfonyl)ethylamino)methyl)furyl;
R₃ is chosen from hydrogen, halogen, trifluoromethyl, cyano, ethynyl, nitro, amino, hydroxyl, hydroxymethyl, methanol methanesulfonic acid ester, methoxyl, acetamido, benzamido, morpholino, 3-fluoro-benzyloxy;
R₄ is chosen from hydrogen, methyl, trifluoromethyl, n-butyl, Methoxyethyl;
R₅ is chosen from hydrogen, halogen, trifluoromethyl, cyano, ethynyl, nitro, amino, hydroxyl, hydroxymethyl, methanol methanesulfonic acid ester, methoxyl, acetamido, benzamido, morpholino, 3-fluoro-benzyloxy;
R₁, R₂, R₃, R₄, R₅ are not simultaneously hydrogen.

In one embodiment, the active pharmaceutical ingredient as disclosed herein is chosen from polycyclic quinazolines as listed below:
V-1 9-chloro-2,3-bis(2-methoxyethoxy)-8H-quinazolino[4,3-b]quinazoline hydrochloride monohydrate,
V-1' 9-chloro-2,3-bis(2-methoxyethoxy)-8H-quinazolino[4,3-b]quinazoline,
V-2 9-bromo-2,3-bis(2-methoxyethoxy)-8H-quinazolino[4,3-b]quinazoline hydrochloride hemihydrate,
V-2' 9-bromo-2,3-bis(2-methoxyethoxy)-8H-quinazolino[4,3-b]quinazoline,
V-3 9-fluoro-2,3-bis(2-methoxyethoxy)-8H-quinazolino[4,3-b]quinazoline mesylate,
V-3' 9-fluoro-2,3-bis(2-methoxyethoxy)-8H-quinazolino[4,3-b]quinazoline,
V-4 9-ethynyl-2,3-bis(2-methoxyethoxy)-8H-quinazolino[4,3-b]quinazoline hydrochloride trihydrate,
V-4' 9-ethynyl-2,3-bis(2-methoxyethoxy)-8H-quinazolino[4,3-b]quinazoline,
V-5 9-nitro-2,3-bis(2-methoxyethoxy)-8H-quinazolino[4,3-b]quinazoline,
V-6 9-amino-2,3-bis(2-methoxyethoxy)-8H-quinazolino[4,3-b]quinazoline hydrobromide trihydrate,
V-6' 9-amino-2,3-bis(2-methoxyethoxy)-8H-quinazolino[4,3-b]quinazoline,
V-7 9-cyano-2,3-bis(2-methoxyethoxy)-8H-quinazolino[4,3-b]quinazoline,
V-8 9-hydroxy-2,3-bis(2-methoxyethoxy)-8H-quinazolino[4,3-b]quinazoline,
V-9 9-trifluoromethyl-2,3-bis(2-methoxyethoxy)-8H-quinazolino[4,3-b]quinazoline,
V-10 9-methoxy-2,3-bis(2-methoxyethoxy)-8H-quinazolino[4,3-b]quinazoline,
V-11 N-{2,3-bis(2-methoxyethoxy)-8H-quinazolino[4,3-b]quinazoline}acetamide,
V-12 N-{2,3-bis(2-methoxyethoxy)-8H-quinazolino[4,3-b]quinazoline}benzamide,
V-13 9-chloro-10-fluoro-2,3-bis(2-methoxyethoxy)-8H-quinazolino[4,3-b]quinazoline,
V-14 9-chloro-10-(3-fluoro-benzyloxy)-2,3-bis(2-methoxyethoxy)-8H-quinazolino[4,3-b]quinazoline,
V-15 11-chloro-2,3-bis(2-methoxyethoxy)-8H-quinazolino[4,3-b]quinazoline,
V-16 11-fluoro-2,3-bis(2-methoxyethoxy)-8H-quinazolino[4,3-b]quinazoline,
V-17 11-cyano-2,3-bis(2-methoxyethoxy)-8H-quinazolino[4,3-b]quinazoline,
V-18 11-hydroxy-2,3-bis(2-methoxyethoxy)-8H-quinazolino[4,3-b]quinazoline,
V-19 11-ethynyl-2,3-bis(2-methoxyethoxy)-8H-quinazolino[4,3-b]quinazoline,
V-20 mesylate-{9-nitro-2,3-bis(2-methoxyethoxy)-8H-quinazolino[4,3-b]quinazoline-11-methanol}ester hydrochloride,
V-20' mesylate-{9-nitro-2,3-bis(2-methoxyethoxy)-8H-quinazolino[4,3-b]quinazoline-11-methanol}ester,
V-21 9-amino-11-hydroxymethyl-2,3-bis(2-methoxyethoxy)-8H-quinazolino[4,3-b]quinazoline,
V-22 11-chloro-8-methyl-2,3-bis(2-methoxyethoxy)-8H-quinazolino[4,3-b]quinazoline malate,
V-22' 11-chloro-8-methyl-2,3-bis(2-methoxyethoxy)-8H-quinazolino[4,3-b]quinazoline,
V-23 4-(3-((9-chloro-10-fluoro-3-methoxy-8H-quinazolino[4,3-b]quinazoline-2-yl)oxy)propyl)morpholine di-p-toluenesulfonate trihydrate,
V-23' 4-(3-((9-chloro-10-fluoro-3-methoxy-8H-quinazolino[4,3-b]quinazoline-2-yl)oxy)propyl)morpholine,
V-24 9-chloro-3-methoxy-2-(3-(pyrrolidine-1-yl)propoxy)-8H-quinazolino[4,3-b]quinazoline,
V-25 9-fluoro-3-methoxy-2-(3-(piperazinyl-1-yl)propoxy)-8H-quinazolino[4,3-b]quinazoline,
V-26 4-(3-((9-ethynyl-3-methoxy-8H-quinazolino[4,3-b]quinazoline-2-yl)oxy)propyl)morpholine,
V-27 3-methoxy-2-(3-morpholinyl propoxy)-8H-quinazolino[4,3-b]quinazoline-9-nitrile,
V-28 4-(3-((11-chloro-3-methoxy-8H-quinazolino[4,3-b]quinazoline-2-yl)oxy)prop-yl)morpholine,
V-29 mesylate-{3-(((9-nitro-8H-quinazolino[4,3-b]quinazoline-2-yl)oxy)propyl)m-orpholine-11-methanol}ester,
V-30 9-chloro-10-fluoro-2,3-dimethoxy-8H-quinazolino[4,3-b]quinazoline,
V-31 9-ethynyl-2,3-dimethoxy-8H-quinazolino[4,3-b]quinazoline,
V-32 9-cyano-2,3-dimethoxy-8H-quinazolino[4,3-b]quinazoline,
V-33 N-((5-(9-chloro-10-(3-fluorobenzyloxy)-8H-quinazolino[4,3-b]quinazoline-2-yl)furan-2-yl)methyl)-2-(methylsulfonyl)ethylamine,
V-34 N-((5-(9-chloro-10-fluoro-8H-quinazolino[4,3-b]quinazoline-2-yl)furan-2-yl)methyl)-2-(methylsulfonyl)ethylamine,
V-35 N-((5-(9-ethynyl-8H-quinazolino[4,3-b]quinazoline-2-yl)furan-2-yl)methyl)-2-(methylsulfonyl)ethylamine,
V-36 N-((5-(11-chloro-8H-quinazolino[4,3-b]quinazoline-2-yl)furan-2-yl)methyl)-2-(methylsulfonyl)ethylamine,
V-37 11-chloro-8-n-butyl-2,3-bis(2-methoxyethoxy)-8H-quinazolino[4,3-b]quinazoline,
V-38 11-chloro-2,3-bis(2-methoxyethoxy)-8-(2-methoxyethyl)-8H-quinazolino[4,3-b]quinazoline,
V-39 2-chloro-11-cyano-8H-quinazolino[4,3-b]quinazoline,
V-40 11-chloro-2-nitro-8H-quinazolino[4,3-b]quinazoline,
V-41 11-chloro-2-amino-8H-quinazolino[4,3-b]quinazoline,
V-42 11-chloro-2-methoxy-3-trifluoromethyl-8H-quinazolino[4,3-b]quinazoline,
V-43 11-chloro-2-(3-fluoro-benzyloxy)3-methoxy-8H-quinazolino[4,3-b]quinazoline,
V-44 9-bromo-3-methoxy-2-n-propyl-8H-quinazolino[4,3-b]quinazoline,
V-45 9-bromo-8H-quinazolino[4,3-b]quinazoline-2- formic acid ethyl ester,
V-46 (E)-(9-bromo-8H-quinazolino[4,3-b]quinazoline-2-yl)-4(dimethylamino)-2-butene amide hydrochloride,
V-46'(E)-(9-bromo-8H-quinazolino[4,3-b]quinazoline-2-yl)-4(dimethylamino)-2-butene amide,
V-47 9-bromo-8H-quinazolino[4,3-b]quinazoline-2-formic acid,
V-48 4-(2,3-dimethoxy-8H-quinazolino[4,3-b]quinazoline-9-yl)morpholine,
V-49 11-chloro-2,3-dimethoxy-8-trifluoromethyl-8H-quinazolino[4,3-b]quinazoline.

The above listes active pharmaceutical ingredient denoted as V1 to V-49 are illustrated by chemical structures below:

| Number | Chemical structure | Number | Chemical structure |
|---|---|---|---|
| V-1 | 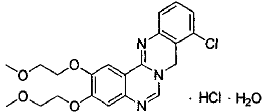 | V-26 | 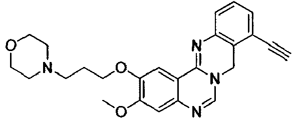 |
| V-2 | 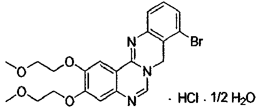 | V-27 | 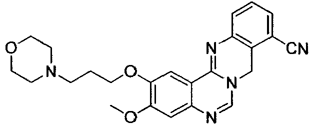 |
| V-3 | 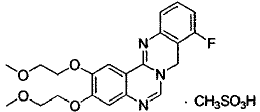 | V-28 | 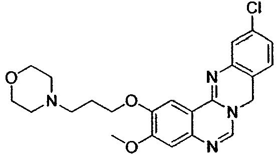 |
| V-4 | 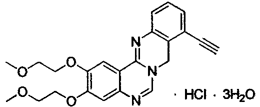 | V-29 | 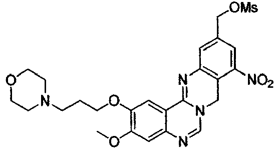 |
| V-5 | 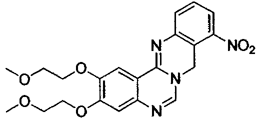 | V-30 | 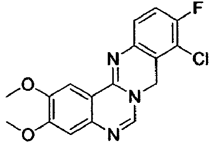 |
| V-6 | 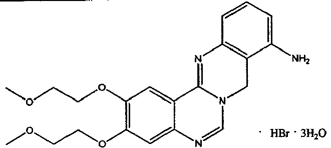 | V-31 | 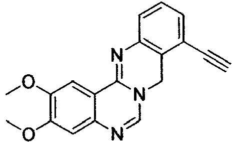 |
| V-7 | 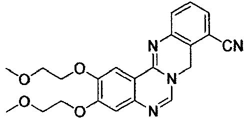 | V-32 | 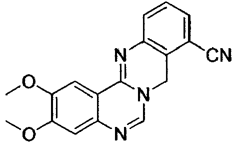 |
| V-8 | 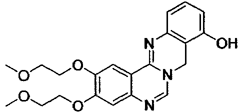 | V-33 | 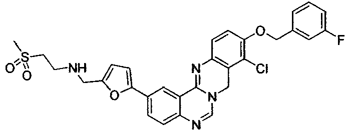 |
| V-9 | 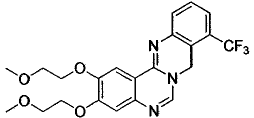 | V-34 | 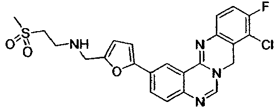 |
| V-10 | 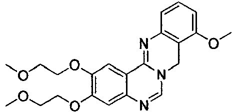 | V-35 | 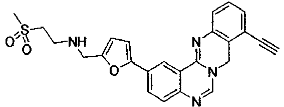 |
| V-11 | 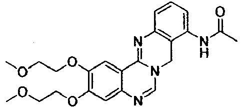 | V-36 | 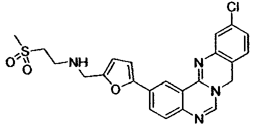 |
| V-12 | 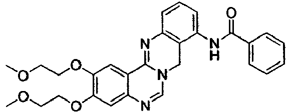 | V-37 | 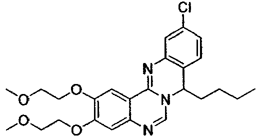 |
| V-13 | 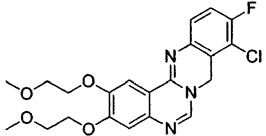 | V-38 | 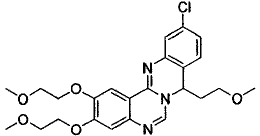 |
| V-14 | 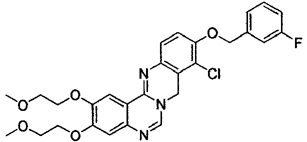 | V-39 | 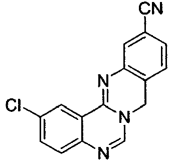 |
| V-15 | 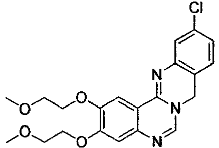 | V-40 | 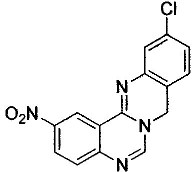 |
| V-16 | 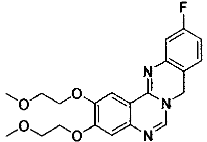 | V-41 | 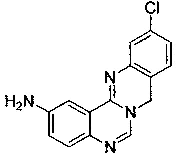 |
| V-17 | 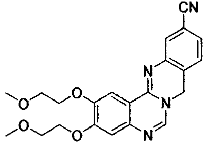 | V-42 | 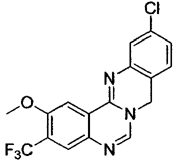 |
| V-18 | 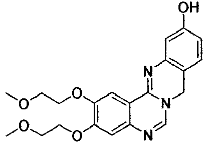 | V-43 | 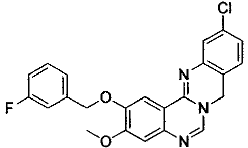 |
| V-19 | 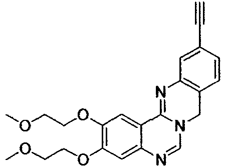 | V-44 | 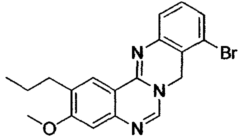 |
| V-20 | 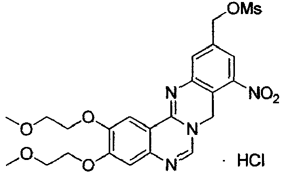 | V-45 | 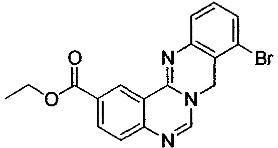 |
| V-21 | 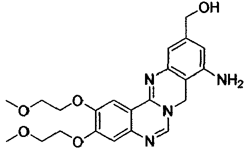 | V-46 | 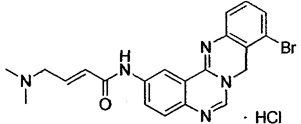 |
| V-22 | 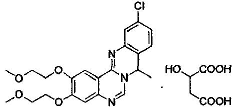 | V-47 | 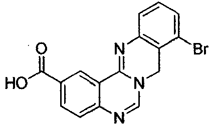 |
| V-23 | 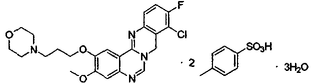 | V-48 | 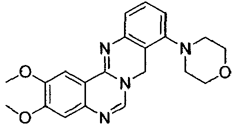 |
| V-24 | 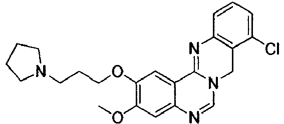 | V-49 | 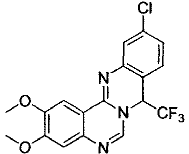 |
| V-25 | 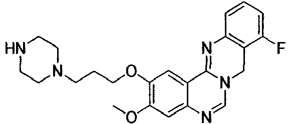 | | |

The above active pharmaceutical ingredient denoted as V-1'to V-4', V-6',V-20',V-22',V-23'and V-46' are illustrated by chemical structures below:

| | | | |
|---|---|---|---|
| V-1' | 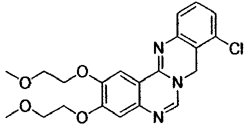 | V-20' | 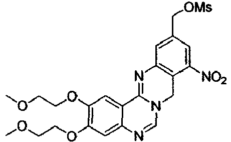 |
| V-2' | 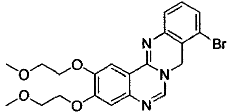 | V-22' | 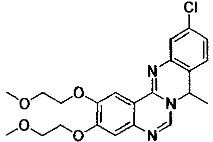 |
| V-3' | 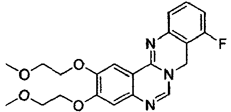 | V-23' | 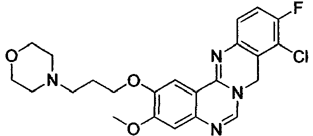 |
| V-4' | 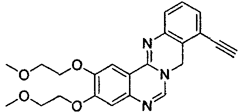 | V-46' | 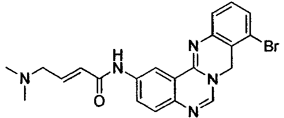 |
| V-6' | 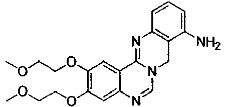 | | |

The polycyclic quinazolines, salts thereof, and hydrates of the salts can be synthesized by the general and exemplary methods below.

### General Method I (Scheme I)

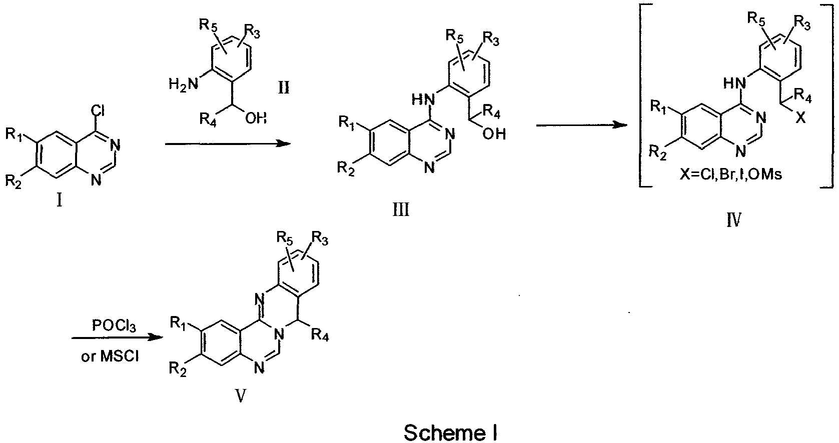

The general method I as shown in scheme I includes steps as following:
(I)the reagents denoted as formula I and formula II are dissolved in solvent A to form a solution,
(II) the reagents base A or acid B is then added to the solution formed in step I, obtain the compound denoted as formula III from the reaction mixture,
(III) the compound denoted as formula V is obtained by reacting the formula III with POCl₃ or methanesulfonyl chloride (MsCl) in solvent B.

The concrete step of scheme I as follow:

The reagents denoted as formula I (10 mmol) and formula II (11 mmol) above are dissolved in solvent A to form a solution; the reagents base A (2 mmol) or acid B (2 mmol) is then added dropwise to the solution. The reaction is stopped after stirring the solution for 3-7 h at room temperature. The reaction mixture is then filtered to obtain a precipitate. The precipitate is dried to obtain intermediate III.

Intermediate III is dissolved in solvent B to obtain a solution. POCl₃ or methanesulfonyl chloride (MsCl) is added dropwise to the solution. The reaction reacts at a temperature ranging from room temperature to the reflux state over 1-3 h. The unreacted reagents are evaporated, and the residue is poured into ice water. The pH of the ice water containing the residue is adjusted to about 10 with saturated Na₂CO₃ solution. The ice water containing the residue is extracted by ethyl acetate for three times, and the organic layers are combined. The combined organic layers are washed with water, then with saturated NaCl solution successively. After being dried by anhydrous sodium sulfate and then filtered, the filtrate is concentrated under vacuum to obtain the crude product as brown oil or solid. The crude product is recrystallized or purified by flash column chromatography to obtain product V. Product V is dissolved in a solution of acid in alcohol solution (3 mol/L) at elevated temperature, and then the solution is cooled down to the temperature to obtain a precipitate, which is a salt of compound V.

The acid as described above is chosen from hydrochloric acid, hydrobromic acid, sulfuric acid, acetic acid, lactic acid, tartaric acid, tannic acid, citric acid, trifluoroacetic acid, malic acid, maleic acid, succinic acid, p-toluenesulfonic acid, and methanesulfonic acid. Thus the salt obtained is correspondingly hydrochloride, hydrobromide, sulfate, acetate, lactate, tartrate, tannic acid salt (tannate), citric acid salt (citrate), trifluoroacetic acid salt (trifluoroacetate), malate, maleate, succinate, p-toluenesulfonate, or mesylate.

Solvent A mentioned above is chosen from acetonitrile, isopropyl alcohol, dichloromethane, N,N-dimethylformamide, N,N-dimethylacetamide, and 1,4-dioxane. Solvent B mentioned above is chosen from acetonitrile, dichloromethane, chloroform, toluene, and benzene. Base A can be chosen from triethylamine, pyridine, potassium carbonate, and diisopropyl ethylamine. Acid B can be chosen from formic acid, acetic acid, hydrochloric acid, sulfuric acid, and phosphoric acid.

In the scheme I, wherein, R₁ is chosen from hydrogen, halogen, amino, nitro, hydroxamino, carboxyl, C₁-C₄ alkoxy carbonyl, C₁-C₄ alkyl, C₁-C₄ acylamino , (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₂ is chosen from hydrogen, halogen, amino, nitro, hydroxamino, carboxyl, C₁-C₄ alkoxy carbonyl, C₁-C₄ alkyl, C₁-C₄ acylamino , (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₃ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic, substituted benzyloxy;
R₄ is chosen from hydrogen, trifluoromethyl, C₁-C₄ alkyl and C₁-C₄ alkoxy;
R₅ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic, substituted benzyloxy;
R₁, R₂, R₃, R₄ and R₅ are not simultaneously hydrogen.

Reagent I can be synthesized according to the method disclosed in WO9630347 and Tetrahedron Letters, 2004 (45), 6517-6521 (see Scheme II). Reagent II can be synthesized according to the method disclosed in J. Med. Chem.1986, 29. 1406-1412 and WO patent No. 2005067933A (see Scheme III).

### General method 2 (Scheme II)

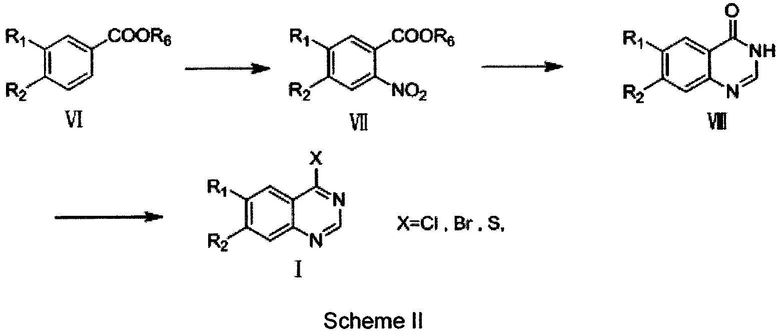

R₁ and R₂ as shown in Scheme II are the same as defined for formula V in Scheme I. R6 can be chosen from hydrogen, methyl, ethyl, and isopropyl.

Compound VI (0.05 mol) is dissolved in glacial acetic acid 50 ml. The resulting solution is stirred in an ice bath, then 13 ml of nitric acid (65-68% by weight) is added into the glacial acetic acid solution. The reaction solution is stirred for 24 h at room temperature, then the reaction solution is poured into 500 ml of ice water, and extracted by ethyl acetate three times. The organic layer is combined, washed with saturated NaHSO₄ solution three times, then washed with saturated sodium chloride solution, and dried by anhydrous sodium sulfate. The solution dried by the anhydrous sodium sulfate is then filtered, and the filtrate is concentrated to obtain intermediate VII.

Compound VII (0.10 mol), ammonium formate (63 g, 1.00 mol), 5% Pd/C (5.00 g), and formamide (75 ml) are reacted at 150°C for 6.5 h. The reaction mixture is cooled to room temperature and allowed to precipitate. Then the reaction mixture is filtered to obtain a white solid compound VIII.

Compound VIII (0.15 mol) is dissolved in the mixture of N,N-dimethylformamide (1 ml) and dichloromethane (150 ml). The resulting solution is stirred at room temperature, while thionyl chloride (14.9 g) (or oxalyl chloride or phosphorus oxychloride) is added dropwise to the solution. The solution is then refluxed for 6 h, the reaction mixture is cooled to room temperature, and the pH value is adjusted to 7-8 by sodium hydroxide solution. The reaction mixture is left to settle. The organic phase of the reaction mixture is collected and concentrated under vacuum to obtain compound I.

General method III (Scheme III)

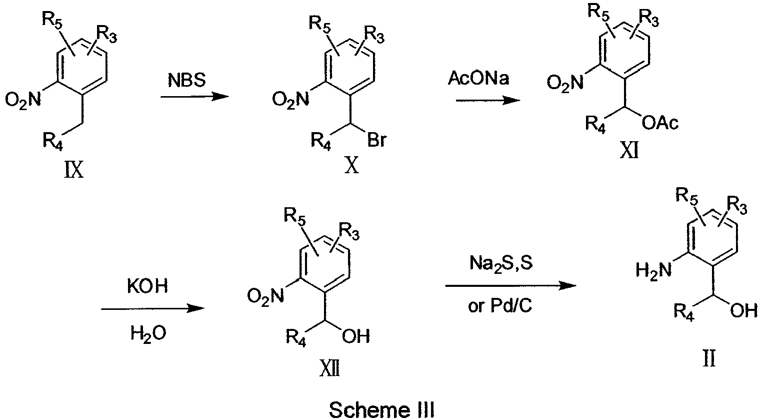

The compound IX (0.20 mol), N-bromosuccinimide (26.6 g, 0.20 mol), and benzoyl peroxide (0.40 g, 2 mmol) are dissolved in 100 ml chlorobenzene (or carbon tetrachloride), heated and refluxed for 20 h. After the reaction, the solution is filtered to obtain a filtrate. The filtrate is concentrated under vacuum to obtain a light brown oily substance, which is then recrystallized in ethanol or purified by silica gel column chromatography to obtain the compound X.

Compound X (0.05 mol) and anhydrous sodium acetate (14.7 g, 0.15 mol) are added into 70 ml N,N-dimethylformamide, heated to 70°C and reacted for 1 h, then the solution is cooled down to room temperature. 150 ml water is added to the solution, and then the solution is extracted by ethyl acetate three times, the organic phase is combined, washed with water three times, then washed with saturated sodium chloride solution three times, and dried by anhydrous sodium sulfate. The solution is then filtered to obtain a filtrate, which is concentrated under vacuum to obtain a white solid compound XI.

Compound XI (0.01 mol) is dissolved into 100 ml water to form a solution, 20 ml of potassium hydroxide aqueous solution (10% by weight) is then added dropwise to the solution. The solution is heated to reflux for 2 h. After the reaction, the mixture is extracted by ethyl acetate three times. The organic layers are combined, washed with water and saturated sodium chloride solution sequentially, and dried by anhydrous sodium sulfate. The reaction mixture is then filtered, and the filtrate is concentrated under vacuum to obtain compound XII.

Compound XII (5 mmol), sodium sulfide (0.78 g, 10 mmol), and sulfur (0.32 g, 10 mmol) are dissolved in the mixture of 50 ml ethanol and 25 ml water, the solution is then heated to reflux for 2 h. The reaction mixture is cooled down to room temperature, and the ethanol is evaporated under vacuum. The reaction mixture is then added into 30 ml water, and extracted by ethyl acetate. The organic phase is combined, washed with water and saturated sodium chloride solution, and dried by anhydrous sodium sulfate. The reaction mixture is then filtered to obtain a filtrate, which is concentrated under vacuum to obtain compound II.

R₃, R₄, and R₅ as shown in Scheme III are the same as defined for Formula V.

Compounds VI and IX can be purchased through commercial sources or synthesized by conventional methods.

Pharmacological tests have shown that the active pharmaceutical ingredient disclosed herein not only demonstrate inhibition on epidermal growth factor receptor tyrosine kinase and aurora kinase, but also can induce cells differentiation and have anti-proliferative activity on certain tumor cells. The active pharmaceutical ingredient disclosed herein can be used for preparation of a medicament for treating cancer and/or the diseases caused by cell abnormal differentiation and/or proliferation.

The disclosure also relates to a composition comprising a therapeutically effective amount of the active pharmaceutical ingredient described herein and at least one pharmaceutically acceptable carrier. The pharmaceutically acceptable carrier may be chosen from bespices, sweeteners, liquid, solid filler, diluent, and other commonly used carrier material. The composition can be made into a common pharmaceutical dosage form by using methods known in the field, such as tablets, capsules, powder, syrup, liquid, suspension, or injection. The weight percentage of active pharmaceutical ingredient disclosed herein in the composition may range from about 1% to about 70%, for example, from about 5% to about 50%.

The active pharmaceutical ingredient disclosed herein can be administered to mammals (included humans) by oral administration or injection. For example, the active pharmaceutical ingredient disclosed herein can be orally administered. Daily dosage can range from 0.0001 to 200 mg/kg by body weight. The optimal dosage may depend on the individual physiological condition. For example, the administration can start with a lower dosage, and then the dosage can increase gradually.

The animal experiments show that the active pharmaceutical ingredient disclosed herein has very low toxicity.

Compared to the existing compounds with similar structures, the active pharmaceutical ingredient disclosed herein show advantages as follow:
(I)the active pharmaceutical ingredient disclosed herein demonstrate inhibition both on epidermal growth factor receptor tyrosine kinase and aurora kinase.
(II)the active pharmaceutical ingredient disclosed herein show strong anti-proliferative activity on certain tumor cells. Specially, the active pharmaceutical ingredient disclosed herein show better anti-proliferative activity and greater inhibitory spectrum against tumor cells than that of erlotinib group.
(III)the active pharmaceutical ingredient disclosed herein have low toxicity and side-effect. Moreover, the active pharmaceutical ingredient disclosed herein have good druggability.
(IV)the active pharmaceutical ingredient disclosed herein and pharmaceutical preparations thereof show good effect on diseases caused by the gene abnormal expression, such as tumor, endocrine disorder, immune system diseases, hereditary disease, nerve system disease.

### Detailed examples

The present disclosure is illustrated further with the following non-limiting examples. Percentage as mentioned below means weight percentages unless otherwise specified.

### EXAMPLE 1

### 9-chloro-2,3-bis(2-methoxyethoxy)-8H-quinazolino[4,3-b]quinazolinehydrochloride monohydrate (V-1)

4-chloro-6,7-bis(2-methoxyethoxy)quinazoline(3.12 g, 10 mmol) (synthesized according to the General Method II) and 2-amino-6-chloro-benzyl alcohol (1.57 g, 10 mmol) (synthesized according to General Method III) were dissolved in 20 ml isopropanol, and heated to reflux for about 4 h. A white solid precipitated. The precipitate was filtered to obtain the crude product, which was recrystallized by ethanol and dried to obtain a white solid intermediate M-1 (4.11 g, yield 87.63%).

M-1 (2.00 g, 4.26 mmol) was dissolved in 3 ml POCl₃, and heated to reflux for about 2 h. The unreacted POCl₃ was evaporated, and the residue was poured into ice water, the pH of the mixture was adjusted to 10 by saturated sodium carbonate (Na₂CO₃) solution, and the mixture was extracted by ethyl acetate three times. The organic layer was combined, washed with water and saturated sodium chloride (NaCl) solution sequently, and dried by anhydrous sodium sulfate. The organic phase was filtered, and the filtrate was concentrated under vacuum to obtain a crude product as a light yellow solid. The crude product was purified by column chromatography to obtain a white solid V-1' (1.33 g, 72.28%).

The white solid V-1' obtained above was dissolved in 3 mol/L hydrochloric acid/ethanol (10 ml) and heated until it was fully dissolved. It was then cooled and precipitated to obtain 1.2 g of the compound V-1, which contains 1 equivalent of solvated water as shown by elemental analysis.

MS (ESI): [M+H]⁺=416.

¹H-NMR (400 MHz,CDCl₃) δppm: 3.50(s, 6H), 3.82(m, 4H), 4.22(t, J=8.4 Hz, 2H), 4.30(t, J=8.4 Hz, 2H), 5.59(s, 2H), 7.10(s, 1 H), 7.21 (m, 2H), 7.39(m, 1H), 7.90(s, 1 H), 8.40(s, 1 H).

### EXAMPLE 2

### 9-bromo-2,3-bis(2-methoxyethoxy)-8H-quinazolino[4,3-b]quinazoline hydrochloride hemihydrates (V-2)

4-chloro-6,7-bis(2-methoxyethoxy)quinazoline (3.12 g, 10 mmol) and 2-amino-6-bromobenzyl alcohol (2.01 g, 10 mmol) (synthesized according to General Method III) were dissolved in 20 ml acetonitrile to form a solution, and 0.2 ml pyridine was added dropwise to the solution. The reaction mixture was heated to reflux for about 4 h, and then a white solid precipitated. The white solid was filtered to obtain a crude product, which was then recrystallized by ethanol and dried to obtain a white solid intermediate M-2 (4.56 g, 88.89%).

M-2 (2.00 g, 3.90 mmol) was dissolved in 3 ml POCl₃, and heated to reflux for about 2 h. The unreacted POCl₃ was evaporated, and the residue was added into ice water. The pH was adjusted to about 9 by saturated sodium carbonate (Na₂CO₃) aqueous solution. The residue was extracted by ethyl acetate three times. The organic phase was combined, washed with water and saturated sodium chloride (NaCl) solution, and dried by anhydrous sodium sulfate. The organic phase was then filtered to obtain a filtrate, which was concentrated under vacuum to obtain a light yellow solid crude product, which was separated by column chromatography to obtain a white solid V-2' (1.21 g, 67.59%).

The white solid V-2' obtained above was dissolved in 3 mol/L hydrochloric acid/ethanol (10 ml) and heated until fully dissolved, and then the solution was cooled and precipitated to obtain 1.02 g of the compound V-2. This salt was shown to contain 0.5 equivalent of solvated water by elemental analysis.

MS (ESI): [M+H]⁺=460.

¹H-NMR (400 MHz,CDCl₃) δppm: 3.50(s, 6H), 3.85(m, 4H), 4.20(m, 2H), 4.28(m, 2H), 5.32(s, 2H), 7.05-7.10(m, 2H), 7.20(m, 1 H), 7.30(s, 1H), 7.75(s, 1 H), 8.02(s, 1 H).

### EXAMPLE 3

### 9-fluoro-2,3-bis(2-methoxyethoxy)-8H-quinazolino[4,3-b]quinazoline mesylate (V-3)

4-chloro-6,7-bis(2-methoxyethoxy)quinazoline (3.12 g, 10 mmol) and 2-amino-6-fluorobenzyl alcohol (1.41 g, 10 mmol) were dissolved in 20 ml isopropanol to form a solution, and 0.55 ml of concentrated hydrochloric acid was added dropwise to the solution. The reaction was carried out according to General Method I to obtain a white solid intermediate M-3 (3.92 g, 85.96%).

M-3 (2.27 g, 5 mol) was dissolved in 15 ml toluene, and cooled in an ice bath. Triethylamine (15 mmol) and methanesulfonyl chloride (20 mmol) were added dropwise to the reaction mixture sequentially. The reaction lasted for 7 h at room temperature. Water (30 ml) was added to the reaction mixture, and the reaction mixture was extracted by dichloromethane three times. The organic phase was combined, washed with water three times, then washed with saturated NaCl solution three times, and dried by anhydrous sodium sulfate. The solution was filtered to obtain a filtrate, which was concentrated under vacuum to obtain the crude product as a light yellow solid, which was recrystallized by ethanol or separated by column chromatography to obtain a white solid V-3' (1.76 g, 88.22%).

The white solid V-3' (2 mmol) obtained above was dissolved in hot ethanol, and methanesulfonic acid (2 mmol) was added dropwise into the ethanol solution. The solution was refluxed for 30 min, cooled and precipitated to obtain the compound V-3.

MS (ESI):[M+H]⁺=400.

¹H-NMR (400 MHz,CDCl₃) δppm: 3.44(s, 6H), 3.85(m, 4H), 4.26(m, 2H), 4.41(m, 2H), 5.61(s, 2H), 7.12(s, 1H), 7.18-7.30(m, 3H), 7.6(8s, 1 H), 8.32(s, 1 H).

### EXAMPLE 4

### 9-ethynyl-2,3-bis(2-methoxyethoxy)-8H-quinazolino[4,3-b]quinazoline hydrochloride trihydrate (V-4)

V-2 (5.07 g, 11 mmol), 2-methyl-3-butyn-2-ol (1.00 g, 10 mmol), palladium acetate (0.006 g, 0.025 mmol), copper iodide (0.012 g, 0.05 mmol) and triphenylphosphine (0.08 g, 0.3 mmol) were dissolved in 10 ml triethylamine, and refluxed for about 7 h under N₂. The reaction mixture was cooled to room temperature, and filtered to remove the insoluble substance. The filtrate was concentrated under vacuum to obtain a brown oily substance. The oily substance was dissolved in ethyl acetate (30 ml), washed with aqueous solution of EDTA-2Na three times, with water three times, and with saturated NaCl solution three times. The organic layer was collected, and concentrated under vacuum to obtain a brown oil, which was recrystallized by isopropanol and dried to obtain a gray solid intermediate M-4 (3.34 g, 65.58%).

M-4 (1.00 g, 2 mmol) and NaOH (0.05 g, 1.25 mmol) were dissolved in toluene (30 ml), and heated to reflux for about 6 h under N₂. The solution was cooled to room temperature, and washed with water 3 times. The organic layer was collected, and concentrated under vacuum to obtain a residue. The residue was subject to flash column chromatography, using a mobile phase of CHCl₂ and EtOAc (1:10, V/V) to obtain a gray solid V-4' (0.35 g, 43.21%).

The gray solid V-4' obtained above was dissolved in a hot solution of HCl/Ethanol (3 mol/L, 10 ml), cooled and precipitated to obtain the compound V-4 (0.23 g). This V-4 was shown to contain 3 equivalents of solvated water by elemental analysis.

MS (ESI): [M+H]⁺=406.

¹H-NMR (400 MHz, CDCl₃) δppm: 3.50(s, 6H), 3.80(m, 4H), 4.12(m, 2H), 4.26(m, 2H), 4.26(m, 2H), 4.56(s, 2H), 5.36(s, 2H), 7.05(s, 1H), 7.24-7.38(m, 3H), 7.65(s, 1H), 8.62(s, 1H).

### EXAMPLE 5

### 9-nitro-2,3-bis(2-methoxyethoxy)-8H-quinazolino[4,3-b]quinazoline (V-5)

4-chloro-6,7-bis(2-methoxyethoxy)quinazoline (3.12 g, 10 mmol) and 2-amino-6- nitrobenzyl alcohol (1.68 g, 10 mmol) (synthesized according to General Method III) were dissolved in 1,4-dioxane (20 ml), and concentrated hydrochloric acid (0.15 ml) was added. The reaction was allowed to react for 7 h at room temperature, and a white solid precipitated. The precipitate was filtered to obtain the crude product, which was recrystallized by ethanol and dried to obtain intermediate M-5 as a white solid (4.15 g, 86.46%).

M-5 (2.00 g, 4.16 mmol) was dissolved in CHCl₃ (15 ml), and in an ice water bath, triethylamine (0.5 ml) and methanesulfonyl chloride (0.4 ml) was added sequentially. The reaction was allowed to react for 7-10 h at room temperature. The reaction mixture was then added into water (30 ml) and extracted by CH₂Cl₂ three times. The organic phase was combined, washed sequentially by water three times and saturated NaCl solution three times, and dried by anhydrous sodium sulfate. Then the organic phase was filtered to obtain a filtrate. The filtrate was concentrated under vacuum to obtain a crude compound, which was then recrystallized by alcohol or separated by column chromatography to obtain a white solid V-5 (1.21 g, 68.12%).

MS (ESI): [M+H]⁺=427.

¹H-NMR(400 MHz, CDCl₃) δppm: 3.50(s, 6H), 3.85(m, 4H), 4.30(m, 2H), 4.51(m, 2H), 5.42(s, 2H), 6.82(s, 1 H), 7.19(m, 1 H),7.29(s, 1 H),7.38(m, 1H), 7.58(m, 1 H), 7.65(s, 1 H).

### EXAMPLE 6

### 9-amino-2,3-bis(2-methoxyethoxy)-8H-quinazolino[4,3-b]quinazoline hydrobromide trihydrate (V-6)

V-5 (0.50 g, 1.17 mmol) and 10% Pd/C (0.10 g) were added into methanol (15 ml), heated to reflux, hydrogenated for 5 h under normal pressure, and then filtered. The filtrate was cooled in a freezer for 7 h, and a large amount of white substance precipitated. The precipitate was filtered and dried to obtain a white solid V-6' (0.36 g, 77.70%).

The white solid V-6' obtained above was dissolved in hot ethanol, and 2 ml of HBr/acetic ether solution (4 mol/L) was added. The solution was cooled and precipitated to obtain compound V-6, which was shown to contain 3 equivalents of solvated water by elemental analysis.

MS (ESI): [M+H]⁺=397.

¹H-NMR (400 MHz, DMSO-d6) δppm: 3.45(s, 6H), 3.85(m, 4H), 4.25(m, 2H), 4.40(m, 2H), 5.19(m, 2H), 6.39(s, J=6.4 Hz, 1H), 6.82-6.95(m, 2H), 7.00(s, 1H), 7.68(s, 1H), 8.00(s, 2H).

### EXAMPLE 7

### 9-cyano-2,3-bis(2-methoxyethoxy)-8H-quinazolino[4,3-b]quinazoline (V-7)

4-chloro-6,7-bis(2-methoxyethoxy)quinazoline (3.12 g,10 mmol) and 2-amino-6-cyanobenzyl alcohol (1.63 g, 11 mmol) were dissolved in isopropanol (20 ml), and diisopropylethylamine (0.50 ml) was added. The reaction was carried out according to General Method I for preparing the intermediate III to obtain a white solid intermediate M-6 (3.97 g, 86.30%).

M-6 (2.12 g, 5 mmol) was dissolved in CHCl₃ (15 ml) to form a solution, and in an ice water bath, triethylamine (15 mmol) and methanesulfonyl chloride (20 mmol) were added dropwise sequentially to the solution. The reaction was carried out according to General Method I for preparing compound V to obtain a white solid V-7(1.50 g, 59.17%).

MS(ESI): [M+H]⁺=508.

¹H-NMR (400 MHz, CDCl₃) δppm: 3.50(s, 6H), 3.82(m, 4H), 4.24(m, 2H), 4.33(m, 2H), 5.60(s, 2H), 7.20(s, 1 H), 7.26-7.41 (m, 3H), 7.89(s, 1 H), 8.42(s, 1 H).

### EXAMPLE 8

### 9-hydroxy-2,3-bis(2-methoxyethoxy)-8H-quinazolino[4,3-b]quinazoline (V-8)

4-chloro-6,7-bis(2-methoxyethoxy)quinazoline (3.12 g, 10 mmol) and 2-amino-6-hydroxyl-benzyl alcohol (1.39 g,10 mmol) were dissolved in isopropanol (20 ml), and concentrated hydrochloric acid (0.55 ml) was added dropwise to the solution. The reaction was carried out according to General Method I for preparing the intermediate III to obtain a white solid intermediate M-6 (3.55 g, 78.71%).

M-7 (2.26 g, 5 mmol) was dissolved in CH₂Cl₂ (25 ml) to form a solution. In an ice water bath triethylamine (15 mmol) and methanesulfonyl chloride (20 mmol) were added dropwise sequentially to the solution. The reaction mixture was added into water (30 ml), and extracted by CH₂Cl₂ three times. The organic phase was combined, washed sequentially by water three times, and saturated NaCl solution three times, and then dried by anhydrous sodium sulfate. The organic phase was filtered to obtain a filtrate, which was concentrated under vacuum to obtain a light yellow compound intermediate M-8 (1.72 g, 62.89%).

M-8 (2.38 g, 5 mol) was dissolved in water (30 ml) to form a solution and 10% NaOH (2.0 ml) was added dropwise to the solution. The reaction mixture was refluxed for about 2 h, and then cooled to room temperature. The pH of the reaction mixture was adjusted to 5 by a hydrochloric acid (HCl) solution. A white solid precipitated. The precipitate was filtered to obtain compound V-8 (1.50 g, 75.57%).

MS(ESI): [M+H]⁺=398.

¹H-NMR(400 MHz, CDCl₃) δppm: 3.35(s, 6H), 3.85(m, 4H), 4.20(m, 2H), 4.40(m, 2H), 5.19(m, 2H), 5.51 (s, 1H), 6.92(s,J=8.2 Hz, 1 H), 7.02-7.24(m, 3H), 7.46(s, 1H), 8.05(s, 1 H).

### EXAMPLE 9

### 9-trifluoromethyl-2,3-bis(2-methoxyethoxy)-8H-quinazolino[4,3-b]quinazoline (V-9)

4-chloro-6,7-bis(2-methoxyethoxy)quinazoline (3.12 g, 10 mmol) and 2-amino-6-trifluoromethyl-benzyl alcohol (2.10 g, 11 mmol) were dissolved in N,N-dimethylformamide (20 ml), and K₂CO₃ (4.14 g) was added. The reaction mixture was heated to 80 °C, reacted for about 4 h, and cooled to room temperature. The reaction mixture was poured into cold water (100 ml), and a white solid precipitated. The precipitate was filtered to obtain a crude product. The crude product was recrystallized by 95% ethanol, and dried to obtain a white solid intermediate M-9 (4.17 g, 82.90%).

M-9 (2.52 g, 5 mmol) was dissolved in CHCl₃ (15 ml), and in an ice water bath, triethylamine (15 mmol) and methanesulfonyl chloride (20 mmol) were added dropwise sequentially. The reaction was carried out according to General Method I for preparing the product V to obtain a white solid V-9 (1.66 g, 73.94%).

MS(ESI): [M+H]⁺=450.

¹H-NMR(400 MHz,CDCl₃) δppm: 3.45(s, 6H), 3.80(m, 4H), 4.35(m, 2H), 4.51(m, 2H), 5.32(s, 2H), 6.88(s, 1H), 7.05-7.22(m, 2H), 7.31 (s, 1H), 7.44(m, 1H), 7.82(m, 1H), 8.05(s, 1H).

### EXAMPLE 10

### 9-methoxy-2,3-bis(2-methoxyethoxy)-8H-quinazolino[4,3-b]quinazoline (V-10)

4-chloro-6,7-bis(2-methoxyethoxy)quinazoline (3.12 g, 10 mmol) and 2-amino-6-methoxyl-benzyl alcohol (1.53 g, 10 mmol) were dissolved in acetonitrile (20 ml). The reaction was carried out according to General Method I for preparing intermediate III to obtain a white solid intermediate M-10 (4.01 g, 86.24%).

M-10 (2.33 g, 5 mmol) was dissolved in CH₂Cl₂ (15 ml). In an ice water bath triethylamine (15 mmol) and methanesulfonyl chloride (20 mmol) were added dropwise sequentially. The reaction was carried out according to General Method I for preparing the product V to obtain a white solid V-10 (1.23 g, 59.85%).

MS (ESI): [M+H]⁺=412.

¹H-NMR(400 MHz, CDCl₃) δppm: 3.50(s, 6H), 3.79(s, 3H), 3.84(m, 4H), 4.32(m, 2H), 4.56(m, 2H), 5.28(s, 2H), 6.72(s, 1H), 7.01-7.18(m, 2H), 7.31(s, 1H), 7.41(m, 1H), 7.66(m, 1H), 7.90(s, 1H).

### EXAMPLE 11

### N-{2,3-bis(2-methoxyethoxy)-8H-quinazolino[4,3-b]quinazoline}acetamide (V-11)

V-6 (0.50 g, 1.17 mmol) was dissolved in CHCl₃ (10 ml), and in an ice water bath, acetyl chloride (0.05 mmol) was added dropwise. The reaction mixture was warmed to room temperature, reacted for about 3 h, and water (20 ml) was added. The reaction mixture was then extracted with CH₂Cl₂ three times. The organic phase was combined, washed sequentially with water three times and saturated NaCl solution three times, and dried by anhydrous sodium sulfate. The organic phase was then filtered to obtain a filtrate, which was concentrated under vacuum to obtain a crude product. The crude product was then recrystallized in alcohol and dried to obtain a white solid V-11 (0.41 g, 79.82%).

MS (ESI): [M+H]⁺=439.

¹H-NMR (400 MHz, CDCl₃) δppm: 2.32(s, 3H), 3.55(s, OCH3, 6H), 3.84(m, 4H), 4.35(t, J=8.0 Hz, 2H), 4.49(t, J=8.2 Hz, 2H), 5.18(s, 2H), 6.72(s, 1H), 7.13-7.18(m, 2H), 7.31(s, 1H), 7.46(m, 1H), 7.66(m, 1H), 8.16(s, 1H), 9.01(s, NH, 1H).

### EXAMPLE 12

### N-{2,3-bis(2-methoxyethoxy)-8H-quinazolino[4,3-b]quinazoline}benzamide (V-12)

V-6 (0.50 g, 1.17 mmol) was dissolved in CHCl₃(10 ml), and in an ice bath, benzoyl chloride (0.18 g) was added dropwise. The reaction mixture was warmed to room temperature, and then reacted for about 3 h. Water (20 ml) was then added into the reaction mixture , and extracted with CH₂Cl₂ three times. The organic phase was combined, washed sequentially with water three times and saturated NaCl solution three times, and then dried by anhydrous sodium sulfate. The organic phase was filtered to obtain a filtrate. The filtrate was concentrated under vacuum to obtain a crude product, which was then recrystallized in alcohol and dried to obtain white solid V-12 (0.51 g, 87.18%).

MS (ESI): [M+H]⁺=501.

¹H-NMR(400 MHz, CDCl₃) δppm: 3.50(s, 2×CH₃, 6H), 3.84(m, 4H), 4.32(m, 4H), 5.24(s, 2H), 6.78(s, 1H), 6.85(m, 1H), 7.21-7.33(m, 3H), 7.56(m, 2H), 7.68(m, 1H), 7.80(m, 2H), 8.17(s, 1H), 9.15(s, NH, 1H).

### EXAMPLE 13

### 9-chloro-10-fluoro-2,3-bis(2-methoxyethoxy)-8H-quinazolino[4,3-b]quinazoline (V-13)

4-chloro-6,7-bis(2-methoxyethoxy)quinazoline (3.12 g, 10 mmol) and 2-chloro-3-fluoro-6-amino-benzyl alcohol (1.75 g, 10 mmol) were dissolved in N,N-dimethylacetamide (20 ml), and K₂CO₃ (4.10 g) was added. The reaction mixture was heated to 80□, reacted for about 6 h, and then cooled to room temperature. The reaction mixture was then poured into cold water (100 ml), and a white solid precipitated. The precipitate-containing solution was filtered to obtain a crude product, which was recrystallized by 95% alcohol and dried to obtain a white solid intermediate M-11 (4.11 g, 84.39%).

M-11 (2.44 g, 5 mmol) was dissolved in toluene (15 ml), and in an ice water bath triethylamine (15 mmol) and methanesulfonyl chloride (25 mmol) were added dropwise sequentially. The reaction was carried out according to General Method I for preparing product V to obtain a white solid V-13 (1.55 g, 71.59%).

MS (ESI): [M+H]⁺=434.

¹H-NMR (400 MHz, CDCl₃) δppm: 3.50(s, 6H), 3.85(m, 4H), 4.20(m, 2H), 4.28(m, 2H), 5.32(s, 2H), 7.05-7.10(m, 2H), 7.20(m, 1H), 7.30(s, 1H), 7.75(s, 1H), 8.02(s, 1 H).

### EXAMPLE 14

### 9-chloro-10-(3-fluoro-benzyloxy)-2,3-bis(2-methoxyethoxy)-8H-quinazolino[4-,3-b]quinazoline (V-14)

4-chloro-6,7-bis(2-methoxyethoxy)quinazoline (3.12 g, 10 mmol) and (6-amino-2-chloro-3-((3-fluorophenyl oxy)phenyl)methanol (2.81 g, 10 mmol) were dissolved in isopropanol (30 ml), and HOAc (0.30 ml) was added. The reaction was carried out according to General Method I for preparing the product intermediate III to obtain a white solid intermediate M-12 (4.55 g, 76.72%).

M-12 (3.00 g, 5 mmol) was dissolved in CH₂Cl₂ (15 ml), and in an ice water bath diisopropylethylamine (15 mmol) and methanesulfonyl chloride (25 mmol) were added dropwise sequentially. The reaction was carried out according to General Method I for preparing the product V to obtain a white solid V-14 (1.87 g, 69.39%).

MS (ESI): [M+H]⁺=540.

¹H-NMR(400 MHz, CDCl₃) δppm: 3.50(s, 6H),3.80(m, 4H), 4.25(m, 2H), 4.33(m, 2H), 5.26(s, 2H), 5.87(s, 2H),6.75-6.88(m, 2H), 6.95(m, 1 H), 7.05(m, 1H), 7.20-7.29(m, 2H), 7.40(m, 1H), 7.78(s, 1 H), 8.42(s, 1 H).

### EXAMPLE 15

### 11-chloro-2,3-bis(2-methoxyethoxy)-8H-quinazolino[4,3-b]quinazoline (V-15)

4-chloro-6,7-bis(2-methoxyethoxy)quinazoline (3.12 g, 10 mmol) and 2-amino-4-chlorobenzyl alcohol (1.57 g, 10 mmol) were dissolved in isopropyl alcohol (30 ml), and concentrated hydrochloric acid (0.35 ml) was added dropwise. The reaction was carried out according to General Method I for preparing the intermediate III to obtain a white solid intermediate M-13 (4.05 g, 86.35%).

M-13 (2.35 g, 5 mmol) was dissolved in acetonitrile (15 ml) to form a solution, and in an ice water bath, pyridine (15 mmol) and methanesulfonyl chloride (20 mmol) were added dropwise into the solution sequentially. The reaction was carried out according to General Method I for preparing product V to obtain a white solid V-15 (1.32 g, 63.61%).

MS (ESI): [M+H]⁺=416.

¹H-NMR(400 MHz, CDCl₃) δppm: 3.45(s, 6H), 3.82(m, 4H), 4.25(t, J=8.4 Hz, 2H), 4.32(t, J=8.4 Hz, 2H), 5.46(s, 2H), 6.88(s, 1H), 7.01(m, 1 H), 7.19(s,1H), 7.35-7.42(m, 2H), 8.66(s, 1 H).

### EXAMPLE 16

### 11-fluoro-2,3-bis(2-methoxyethoxy)-8H-quinazolino[4,3-b]quinazoline (V-16)

4-chloro-6,7-bis(2-methoxyethoxy)quinazoline (3.12 g, 10 mmol) and 2-amino-4-fluorobenzyl alcohol (1.41 g, 10 mmol) were dissolved in isopropyl alcohol (30 ml) to form a solution, and concentrated hydrochloric acid (0.55 ml) was added dropwise to the solution. The reaction was carried out according to General Method I for preparing the intermediate III to obtain a white solid intermediate M-14 (3.95 g, 87.20%).

M-14 (2.27 g, 5 mmol) and phosphorus oxychloride (3 ml) were reacted according to General Method I for preparing the product V to obtain a white solid V-16 (1.66 g, 83.21%).

MS (ESI): [M+H]⁺=400.

¹H-NMR (400 MHz, CDCl₃) δppm: 3.50(s, 6H), 3.80(m, 4H), 4.25(t, J=8.0 Hz, 2H), 4.30(t, J=8.0 Hz, 2H), 5.40(s, 2H), 6.76(s, 1H), 7.05-7.20(m, 3H), 7.46(s, 1H), 8.36(s, 1H).

### EXAMPLE 17

### 11-cyano-2,3-bis(2-methoxyethoxy)-8H-quinazolino[4,3-b]quinazoline (V-17)

4-chloro-6,7-bis(2-methoxyethoxy)quinazoline (3.12 g, 10 mmol) and 2-amino-4-cyanobenzyl alcohol (1.63 g, 11 mmol) were dissolved in isopropanol (20 ml) to form a solution, and HOAc (0.30 ml) was added dropwise to the solution. The reaction was carried out according to General Method I for preparing the intermediate III to obtain a white solid intermediate M-15 (3.72 g, 80.87%).

M-15 (2.12 g, 5 mmol) was dissolved in CHCl₃ (15 ml) to form a solution. In an ice water bath triethylamine (15 mmol) and methanesulfonyl chloride (20 mmol) were added dropwise into the solution sequentially. The reaction was carried out according to General Method I for preparing the product V to obtain a white solid V-17 (1.65 g, 65.08%).

MS (ESI): [M+H]⁺=407.

¹H-NMR(400 MHz, CDCl₃) δppm: 3.40(s, 6H), 3.75(m, 4H), 4.20(t, J=7.6 Hz, 2H), 4.28(t, J=8.0 Hz, 2H), 5.62(s, 2H), 6.82(s, 1H), 7.35-7.54(m, 3H), 7.66(s, 1H), 8.15(s, 1H).

### EXAMPLE 18

### 11-hydroxy-2,3-bis(2-methoxyethoxy)-8H-quinazolino[4,3-b]quinazoline (V-18)

According to the procedures of Example 8, 4-chloro-6,7-bis(2-methoxyethoxy)quinazoline (3.12 g,10 mmol) and 2-amino-4-hydroxyl-benzyl alcohol (1.39 g,10 mmol) were used to synthesize and obtain a white solid V-18 (2.03 g, the total yield was 51.13%).

MS (ESI): [M+H]⁺=398.

¹H-NMR (400 MHz, CDCl₃) δppm: 3.45(s, 6H), 3.75(m, 4H), 4.22(t, J=8.0 Hz, 2H), 4.28(t, J=8.0 Hz, 2H), 5.22(s, 2H), 5.46(s, 1 H), 6.67(m, 1 H), 6.78-6.90(m, 2H), 7.00(m, 1H), 7.25(s, 1H), 7.25(s, 1 H), 8.15(s, 1 H).

### EXAMPLE 19

### 11-ethynyl-2,3-bis(2-methoxyethoxy)-8H-quinazolino[4,3-b]quinazoline (V-19)

According to the procedures of Example 4, V-15 (4.56 g, 11 mmol), 2-methyl-3-butyn-2-ol (1.00 g, 10 mmol), palladium acetate (0.006 g, 0.025 mmol), copper iodide (0.012 g, 0.05 mmol), triphenylphosphine (0.08 g, 0.3 mmol), and triethylamine (15 ml) were used to synthesize and obtain a white solid V-19.

MS (ESI): [M+H]⁺=406.

¹H-NMR(400 MHz, CDCl₃) δppm: 3.50(s, 6H), 3.64(m, 4H), 4.20(t, J=8.0 Hz, 2H), 4.32(t, J=8.0 Hz, 2H), 4.80(s, 1H), 5.62(s, 2H), 6.80(s, 1H), 7.26(m, 1H), 7.42-7.58(m, 3H), 8.31 (s, 1H).

### EXAMPLE 20

### mesylate-{9-nitro-2,3-bis(2-methoxyethoxy)-8H-quinazolino[4,3-b]quinazoline-11-methanol}ester hydrochloride (V-20)

4-chloro-6,7-bis(2-methoxyethoxy)quinazoline (3.12 g, 10 mmol) and (2-amino-6-nitro-1,4-phenylene)dimethanol (2.18 g, 11 mmol) were dissolved in N,N-dimethylformamide (20 ml). The reaction was carried out according to General Method I for preparing the intermediate III to obtain a white solid intermediate M-16 (4.33 g, 84.90%).

M-16 (2.55 g, 5 mmol) was dissolved in toluene (25 ml) to form a solution, and in an ice water bath triethylamine (15 mmol) and methanesulfonyl chloride (20 mmol) were added dropwise into the solution sequentially. The reaction was carried out according to General Method I for preparing product V to obtain a white solid V-20' (1.98 g, 74.16%).

The V-20 was prepared by converting the white solid V-20' mentioned above according to the procedures of Example 1.

MS (ESI): [M+H]⁺=535.

¹H-NMR (400 MHz, CDCl₃) δppm: 3.15(s, 3H), 3.52(s, 6H), 3.85(m, 4H), 4.22-4.39(m, 4H), 5.25(s, 2H), 5.65(m, 2H), 7.12(s, 1 H), 7.28(s, 1H), 7.70(s, 1H), 7.75(s, 1 H), 7.82(s, 1H).

### EXAMPLE 21

### 9-amino-11-hydroxymethyl-2,3-bis(2-methoxyethoxy)-8H-quinazolino[4,3-b]quinazoline (V-21)

V-20 (1.00 g, 1.98 mmol) was dissolved in water (20 ml), and 10% KOH solution (1.5 ml) was added dropwise to the V-20 water solution. The reaction mixture was heated to reflux for 1 h, cooled to room temperature, and extracted by EtOAc three times. The organic phase was combined, washed with saturated NaCl solution three times, and then dried by anhydrous sodium sulfate. The organic phase was filtered to obtain a filtrate. The filtrate was concentrated under vacuum to obtain a light yellow solid crude product. The crude product was recrystallized by petroleum ether-EtOAc (5:1, V/V) to obtain a gray solid intermediate M-17 (0.63 g, 69.62%).

M-17 (0.50 g, 1.10 mmol), 0.1 g 10%Pd/C and ethanol (10 ml) were heated to reflux, hydrogenated for 3 h under normal pressure. The reaction mixture was filtered to obtain a filtrate. The filtrate was concentrated under vacuum to obtain a brown oily product, which was recrystallized by ethanol to obtain a white solid V-21 (0.33 g, 70.42%).

MS (ESI): [M+H]⁺=427.

¹H-NMR (400 MHz, CDCl₃) δppm: 3.50(s, 6H), 3.80(m, 4H), 4.35(m, 4H), 4.58(s, 2H), 5.35(s, 2H), 6.55(s, 2H), 6.72(s, 1H), 7.25(s, 1H), 7.64(s, 2H), 8.26(s, 1H).

### EXAMPLE 22

### 11-chloro-8-methyl-2,3-bis(2-methoxyethoxy)-8H-quinazolino[4,3-b]quinazoline malate (V-22)

4-chloro-6,7-bis(2-methoxyethoxy)quinazoline (3.12 g, 10 mmol) and 1- (2-amino-4-chlorophenyl)ethanol (1.88 g, 11 mmol) were dissolved in N,N-dimethylacetamide (20 ml) to form a solution, and potassium carbonate (4.22 g) was added to the solution. The reaction mixture was heated to 80°C and reacted for about 6 h, and cooled to room temperature. The reaction mixture was then poured into cold water (100 ml), and a white solid precipitated. The precipitate was filtered to obtain a crude product, which was recrystallized with 95% ethanol and dried to obtain a white solid intermediate M-18 (4.03 g, 83.44%).

According to General Method I for preparing the product V, M-18 (1.00 g, 2 mmol) and phosphorus oxychloride (3 ml) were used to synthesize and obtain a white solid V-22' (0.45 g, 52.45%).

The white solid V-22' obtained above and malic acid were reacted according to the procedures of example 1 to obtain the V-22 (0.42 g).

MS (ESI): [M+H]⁺=430.

¹H-NMR(400 MHz, CDCl₃) δppm: 2.15(d,J=6.4 Hz, 3H), 3.50(s, 6H, 2×OCH₃), 3.80(m, 4H), 4.28(t, J=8.0 Hz, 2H), 4.31(t, J=8.0 Hz, 2H), 5.12(s, q, 1H), 6.87(s, 1H), 7.11(s, 1H), 7.25-7.32(m, 2H), 7.46(s, 1H), 8.35(s, 1H).

### EXAMPLE 23

### 4-(3-((9-chloro-10-fluoro-3-methoxy-8H-quinazolino[4,3-b]quinazoline-2-yl)-oxy)propyl)morpholine di-p-toluenesulfonate trihydrate (V-23)

4-chloro-7-methoxy-6-(3-morpholino-4-yl-propoxy)quinazoline (3.37 g, 10 mmol) and 3-fluoro-2-chlorine-6-aminobenzyl alcohol (1.75 g, 10 mmol) were dissolved in isopropanol (20 ml) to form a solution, and concentrated hydrochloric acid (0.72 ml) was added dropwise into the solution. The reaction was carried out according to General Method I for preparing the intermediate III to obtain a white solid intermediate M-19 (4.03 g, 78.71%).

According to General Method I for preparing the product V, M-19 (1.00 g, 2 mmol) and phosphorus oxychloride (3 ml) were used to synthesize and obtain a white solid V-23' (0.45 g, 52.45%).

The white solid V-23' obtained above was reacted with p-toluene sulfonic acid to obtain the V-23. The V-23 was shown to contain two equivalents of toluene sulfonate and 3 equivalents of solvated water by elemental analysis.

MS (ESI): [M+H]⁺=459.

¹H-NMR (400 MHz, CDCl₃) δppm: 2.15(m, 2H),2.44-2.52(m, 6H), 3.68(dd, J=8.4 Hz&8.0 Hz, 4H), 3.80(s, 3H), 4.17(t,J=7.6 Hz, 2H), 5.26(s, 2H), 7.02(s, 1H), 7.20(m, 1H), 7.26(s, 1H), 7.34(m, 1H), 7.86(s, 1H), 836(s, 1H).

### EXAMPLE 24

### 9-chloro-3-methoxy-2-(3-(pyrrolidine-1-yl)propoxy)-8H-quinazolino[4,3-b]qu- inazoline (V-24)

4-chloro-7-methoxy-6-(3-pyrrolidino-1-yl)propoxy)quinazoline (3.21 g, 10 mmol) and 2-chloro-6-amino-benzyl alcohol (1.57 g, 10 mmol) were dissolved in CH₂Cl₂ (65 ml) to form a solution, and diisopropyl ethylamine (0.35 ml) was added dropwise into the solution. The reaction was carried out according to General Method I for preparing the intermediate III to obtain a white solid intermediate M-20 (4.13 g, 86.40%).

According to General Method I for preparing product V, M-20 (1.00 g, 2 mmol) and phosphorus oxychloride (3 ml) were used to synthesize and obtain a white solid V-24 (0.40 g, 47.17%).

MS (ESI): [M+H]⁺=425.

¹H-NMR(400 MHz, DMSO-d6) δppm: 2.10(m, 2H), 2.44-2.52(m, 6H), 3.56(dd, J=8.4 Hz&8.6 Hz, 4H), 3.80(s, 3H), 4.22(t, J= 7.8 Hz, 2H), 5.30(s, 2H), 6.90(s, 1H), 7.20-7.35(m, 3H), 7.55(s, 1H), 8.26(s, 1H).

### EXAMPLE 25

### 9-fluoro-3-methoxy-2-(3-(piperazinyl-1-yl)propoxy)-8H-quinazolino[4,3-b]quinazoline(V-25)

According to the procedures of Example 1, 4-chloro-7-methoxy-6-(3-(piperazinyl-1-yl)propoxy)quinazoline (3.36, 10 mmol) and 2-fluoro-6-aminobenzyl alcohol (1.41 g, 10 mmol) were used to synthesize and obtain a white solid V-25 (2.00 g, 47.28%).

MS (ESI): [M+H]⁺=424.

¹H-NMR(400 MHz, DMSO-d6) δppm: 2.12(m, 2H), 2.41-2.47(m, 6H), 3.61(dd, J=8.0 Hz&8.2 Hz, 4H), 3.85(s, 3H), 4.41(t, J=8.0 Hz, 2H), 5.27(s, 2H), 6.95(s, 1H), 7.03-7.22(m, 3H), 7.35 (s, 1H), 8.14(s, 1H).

### EXAMPLE 26

### 4-(3-((9-ethynyl-3-methoxy-8H-quinazolino[4,3-b]quinazoline-2-yl)oxy)propyl)morpholine (V-26)

4-chloro-7-methoxy-6-(3-(morpholino-4-yl-propoxy)quinazoline (3.37 g, 10 mmol) and 2-amino-6-acetylene benzyl alcohol (1.47 g, 10 mmol) were dissolved in isopropanol (20 ml) to form a solution, and concentrated hydrochloric acid (0.65 ml) was added dropwise into the solution. The reaction was carried out according to General Method I for preparing the intermediate III to obtain a white solid intermediate M-21 (4.03 g, 83.78%).

M-21 (2.42 g, 5 mmol) was dissolved in CH₂Cl₂ (25 ml) to form a solution, and in an ice water bath, triethylamine (15 mmol) and methanesulfonyl chloride (25 mmol) were added dropwise into the solution sequentially. The reaction was carried out according to General Method I for preparing the product V to obtain a white solid V-26 (1.38 g, 64.19%).

MS (ESI): [M+H]⁺=431.

1 H-NMR(400 MHz, DMSO-d6) δppm: 2.10(m, 2H), 2.46-2.55(m, 6H), 3.56(dd, J=8.4 Hz&8.0 Hz, 4H), 3.72(s, 3H), 4.20(s, 1H), 4.32(t, J=8.0 Hz, 2H), 5.25(s, 2H), 6.95(s, 1H), 7.208-7.39(m, 3H), 7.66 (s, 1H), 8.45(s, 1H).

### EXAMPLE 27

### 3-methoxy-2-(3-morpholinyl propoxy)-8H-quinazolino[4,3-b]quinazoline-9-nitrile(V-27)

4-chloro-7-methoxy-6-(3-(morpholino-4-yl-propoxy)quinazoline (3.37 g, 10 mmol) and 2-amino-6-nitrile benzyl alcohol (1.48 g, 10 mmol) were dissolved in isopropanol (20 ml) to form a solution, and formic acid (0.40 ml) was added dropwise into the solution. The reaction was carried out according to General Method I for preparing the intermediate III to obtain a white solid intermediate M-22 (4.00 g, 82.47%).

M-22 (2.43 g, 5 mmol) was dissolved in CH₂Cl₂ (25 ml) to form a solution, and in an ice water bath, triethylamine (15 mmol) and methanesulfonyl chloride (25 mmol) were added dropwise into the solution sequentially. According to General Method I for preparing the product V, the reaction was carried out to obtain a white solid V-27 (1.65 g, 76.57%).

MS (ESI): [M+H]⁺=432.

¹H-NMR(400 MHz, DMSO-d6) δppm: 2.10(m, 2H), 2.46-2.55(m, 6H), 3.60(dd, J=8.0 Hz&8.0 Hz, 4H), 3.75(s, 3H), 4.26(s, 1H), 4.35(m, 2H), 5.45(s, 2H), 7.05(s, 1H), 7.19(m,1H), 7.23-7.39(m,2H), 7.56 (s,1H), 8.35(s,1H).

### EXAMPLE 28

### 4-(3-((11-chloro-3-methoxy-8H-quinazolino[4,3-b]quinazoline-2-yl)oxy)propyl)morpholine (V-28)

4-chloro-7-methoxy-6-(3-(morpholino-4-yl-propoxy)quinazoline (3.37 g, 10 mmol) and 2-amino-4-chloro-benzyl alcohol (1.57 g, 10 mmol) were dissolved in isopropanol (20 ml), and concentrated hydrochloric acid (0.65 ml) was added dropwise into the reaction mixture. According to General Method I for preparing the intermediate III, the reaction was carried out to obtain white solid intermediate M-23 (3.90 g, 78.95%).

M-23 (1.48 g, 3 mmol) was dissolved in CH₂Cl₂ (25 ml), and in an ice water bath, triethylamine (10 mmol) and methanesulfonyl chloride (15 mmol) was added dropwise sequentially into the reaction mixture. The reaction was carried out according to General Method I for preparing the product V to obtain a white solid V-28 (0.85 g, 64.39%).

MS (ESI): [M+H]⁺=441.

¹H-NMR(400 MHz, DMSO-d6) δppm: 2.10(m, 2H), 2.43(m, 4H), 2.56(t,J=4.8 Hz), 3.53(dd, J=8.0 Hz&8.0 Hz, 4H), 3.85(s, 3H), 4.26(t, J=7.8 Hz, 2H), 5.42(s, 2H), 7.02(s, 1H), 7.19(m, 1H), 7.26(s, 1H), 7.34-7.39(m, 2H), 8.31 (s, 1H).

### EXAMPLE 29

### mesylate-{3-(((9-nitro--8H-quinazolino[4,3-b]quinazoline-2-yl)oxy)propyl)morpholine-11-methanol}ester (V-29)

4-chloro-7-methoxy-6-(3-(morpholino-4-yl-propoxy)quinazoline (3.37 g, 10 mmol) and 2-amino-6-nitro para-diphenyl methanol (1.48 g, 10 mmol) were dissolved in isopropanol (20 ml), and concentrated hydrochloric acid (0.55 ml) was added dropwise into the reaction mixture. The reaction was carried out according to General Method I for preparing the intermediate III to obtain a white solid intermediate M-24 (4.00 g, 82.47%).

M-24 (2.43 g, 5 mmol) was dissolved in CH₂Cl₂ (25 ml), and in an ice water bath triethylamine (15 mmol) and methanesulfonyl chloride (25 mmol) were added dropwise sequentially into the reaction mixture. The reaction was carried out according to General Method I for preparing the product V to obtain a white solid V-29 (1.65 g, 59.03%).

MS (ESI): [M+H]⁺=560.

¹H-NMR(400 MHz, CDCl₃) δppm: 2.10(m, 2H), 2.45-2.56(m, 6H), 3.30(s, 3H), 3.50(dd, J=8.4 Hz&8.6 Hz, 4H), 3.82(s, 3H), 4.20(t,J=8.0 Hz, 2H), 4.65(s, 2H), 5.31(s, 2H), 6.78(s, 1H), 6.82(s, 1 H), 7.10(s, 1 H), 7.25(s, 1 H), 8.46(s, 1H).

### EXAMPLE 30

### 9-chloro-10-fluoro-2,3-dimethoxy-8H-quinazolino[4,3-b]quinazoline (V-30)

4-chloro-6,7-bis(2-methoxyethoxy)quinazoline (2.24 g, 10 mol) and 2-chloro-3-fluoro-6-amino-benzyl alcohol (1.75 g, 10 mmol) were dissolved in N,N-dimethylformamide (15 ml), and K₂CO₃ (4.22 g) was added to the reaction mixture. The reaction mixture was heated to 80°C and reacted for about 6 h, and cooled to room temperature. The reaction mixture was poured into cold water (100 ml), and a white solid precipitated. The precipitate was filtered to obtain a crude product, which was recrystallized with 95% ethanol and dried to obtain a white solid intermediate M-25 (2.87 g, 71.93%).

According to General Method I for preparing the product V, M-25 (1.20 g, 3 mmol) and phosphorus oxychloride (3 ml) were used to synthesize and obtain a white solid V-30 (0.62 g, 59.90%).

MS (ESI): [M+H]⁺=346.

¹H-NMR(400 MHz, CDCl₃) δppm: 3.70(s, 6H), 5.15(s, 2H), 6.95(s, 1H), 7.05-7.12(m, 2H), 7.55(s, 1H), 8.26(s, 1H).

### EXAMPLE 31

### 9-ethynyl-2,3-dimethoxy-8H-quinazolino[4,3-b]quinazoline

9-iodine-2,3-dimethoxy-8H-quinazoline[4,3-b]quinazoline (4.19 g, 10 mmol), 2-methyl-3-butyn-2-ol (1.00 g, 10 mmol), bis-triphenylphosphine palladium dichloride (0.018 g, 0.026 mmol) and copper iodide (0.012 g, 0.05 mmol) were dissolved in triethylamine (2 ml) and N,N-dimethylformamide (10 ml), and refluxed for about 6 h under N₂. The reaction mixture was cooled to room temperature, and filtered to remove insoluble substance. The filtrate was concentrated under vacuum to obtain a brown oily product. The oily product was dissolved by CH₂Cl₂ (30 ml), washed sequentially with aqueous solution of EDTA-2Na three times, with water three times, and with saturated NaCl solution three times. The organic layer was collected and concentrated under vacuum to obtain a brown oily product. The brown oily product was recrystallized by isopropanol and dried to obtain a gray solid intermediate M-26 (2.76 g, 73.60%).

M-26 (1.00 g, 2.67 mmol) and NaOH (0.06 g, 1.5 mmol) were dissolved in toluene (30 ml) and water (5 ml), heated to reflux for about 6 h under N₂. The reaction mixture was cooled to room temperature, and washed with water three times. The organic layer was collected and concentrated under vacuum to obtain a residue. The residue was separated by flash column chromatography (washed with CH₂Cl₂-EtOAc solution, 1:10, V/V) to obtain a gray solid V-31 (0.71 g, 83.89%).

MS (ESI): [M+H]⁺=318.

¹H-NMR (400 MHz, DMSO-d6) δppm: 3.82 (s, 6H), 4.35 (s, 1H), 5.06 (s, 2H), 6.92 (s, 1H), 7.20 (m, 1 H), 7.33 (s, 1 H), 7.44-7.56 (m, 2H), 8.23 (s, 1H).

### EXAMPLE 32

### 9-cyano-2,3-dimethoxy-8H-quinazolina[4,3-b]quinazoline (V-32)

4-chloro-6,7-bis(2-methoxyethoxy)quinazoline (2.24 g, 10 mol) and 2-amino-6-nitrile-benzyl alcohol (1.48 g, 10 mmol) were dissolved in isopropanol (20 ml), heated to reflux for about 6 h, filtered, and dried under vacuum to obtain a white solid intermediate M-27 (2.97 g, 79.84%).

According to General Method I for preparing the product V, M-27 (1.12 g, 3 mmol) and phosphorus oxychloride (2 ml) were used to synthesize and obtain a white solid V-32 (0.55 g, 57.65%).

MS (ESI): [M+H]⁺=319.

¹H-NMR (400 MHz, CDCl₃) δppm: 3.72(s, 6H), 5.35(s, 2H), 6.90(s, 1H), 7.25(m, 2H), 7.43(s, 1 H), 7.50-7.62(m, 2H), 8.33(s, 1 H).

### EXAMPLE 33

### N-((5-(9-chloro-10-(3-fluoro benzyloxy)-8H-quinazolino[4,3-b]quinazoline-2-yl)furan-2-yl)methyl)-2-(methylsulfonyl)ethylamine (V-33)

N-((5-(4-chloro-quinazoline-6-yl)furan-2-yl)methyl)-2-methylsulfonyl ethylamine (3.65 g, 10 mmol) and (6-amino-2-chloro-3-((3-fluoro-phenyl-oxy)phenyl)methanol (2.81 g, 10 mmol) were dissolved in isopropanol (30 ml), and concentrated hydrochloric acid (0.60 ml) was added dropwise. The reaction was carried out according to General Method I for preparing the intermediate III to obtain a white solid intermediate M-28 (4.75 g, 73.53%).

According to General Method I for preparing the product V, M-28 (2.28 g, 4 mmol) and phosphorus oxychloride (3 ml) were used to synthesize and obtain a white solid V-33 (1.35 g, 57.01%).

MS (ESI): [M+H]⁺=593.

¹H-NMR (400 MHz, DMSO-d6) δppm: 3.00(t, 2H), 3.0 5(s, 3H), 3.36(t, 2H), 3.94(s, 2H), 4.05(s, NH, 1H), 5.26(s, 2H), 5.69(s, 2H), 6.80(d, J=8.8 Hz, 1H), 6.88-6.95(m, 2H), 7.00(s, 1H), 7.05(d, J=8.4 Hz, 1H), 7.18(m, 1H), 7.2 5-7.35(m, 2H), 7.45 (m, 1H), 7.78(m, 1H), 8.16(s, 1H), 8.72(s, 1H).

### EXAMPLE 34

### N-((5-(9-chloro-10-fluoro-8H-quinazolino[4,3-b]quinazoline-2-yl)furan-2-yl- )methyl)-2-(methylsulfonyl)ethylamine (V-34)

N-((5-(4-chloro-quinazoline-6-yl)furan-2-yl)methyl)-2-methylsulfonyl ethylamine (3.65 g, 10 mmol) and 2-chlorine-3-fluoro-6-amino-benzyl methanol (1.93 g, 11 mmol) were dissolved in acetonitrile (30 ml) to form a solution, and concentrated hydrochloric acid (0.55 ml) was added dropwise into the solution. The reaction was carried out according to General Method I for preparing the intermediate III to obtain a white solid intermediate M-29 (4.42 g, 81.85%).

According to General Method I for preparing the product V, M-29 (2.16 g, 4 mmol) and phosphorus oxychloride (5 ml) were used to synthesize and obtain a white solid V-34 (1.26 g, 64.81%).

MS (ESI): [M+H]⁺=487.

¹H-NMR (400 MHz, DMSO-d6) δppm: 3.00(t, 2H), 3.30(s, 3H), 3.45(t, 2H), 3.90(s, 2H), 4.15(s, NH, 1 H), 5.56(s, 2H), 6.90(d, J=8.4 Hz, 1 H), 7.00-7.25(m, 3H), 7.35(m, 1H), 7.48(m, 1 H), 8.01 (s, 1H), 8.35(s, 1H).

### EXAMPLE 35

### N-((5-(9-ethynyl-8H-quinazolino[4,3-b]quinazoline-2-yl)furan-2-yl)methyl)2-(methylsulfonyl)ethylamine (V-35)

N-((5-(4-chloro-quinazoline-6-yl)furan-2-yl)methyl)-2-methylsulfony ethylamine (3.65 g, 10 mmol) and 2-amino-6-ethynyl benzyl alcohol (1.47 g, 10 mmol) were dissolved in acetonitrile (30 ml) to form a solution, and concentrated hydrochloric acid (0.60 ml) was added dropwise to the solution. The reaction was carried out according to General Method I for preparing the intermediate III to obtain a white solid intermediate M-30 (4.00 g, 78.13%).

M-30 (1.54 g, 3 mmol) was dissolved in CH₂Cl₂ (25 ml) to form a solution, and in an ice water bath, triethylamine (9 mmol) and methanesulfonyl chloride (15 mmol) were added dropwise into the solution sequentially. The reaction was carried out according to General Method I for preparing the product V to obtain a white solid V-35 (0.65 g, 47.31%).

MS (ESI): [M+H]⁺=459.

¹H-NMR (400 MHz, DMSO-d6) δppm: 3.10(t, 2H), 3.25(s, 3H), 3.48(t, 2H), 3.90(s, 2H), 4.00(s, 1H), 4.25(s, NH, 1H), 5.35(s, 2H), 6.95(d, J=8.0 Hz, 1H), 7.06(m, 1H), 7.15-7.22(m, 3H), 7.28(m, 1 H), 7.40(m, 1 H), 7.81 (s, 1 H), 8.12(s, 1H).

### EXAMPLE 36

### N-((5-(11-chloro-8H-quinazolino[4,3-b]quinazoline-2-yl)furan-2-yl)methyl)-2-(methylsulfonyl)ethylamine (V-36)

N-((5-(4-chloro-quinazoline-6-yl)furan-2-yl)methyl)-2-methylsulfonyl ethylamine (3.65 g, 10 mmol) and 4-chlorine-2-amino-benzyl alcohol (1.73 g, 11 mmol) were dissolved in isopropanol (30 ml) to form a solution, and concentrated hydrochloric acid (0.60 ml) was added dropwise into the solution. The reaction was carried out according to General Method I for preparing the intermediate III to obtain a white solid intermediate M-31 (4.12 g, 76.58%).

According to General Method I for preparing the product V, M-31 (2.09 g, 4 mmol) and phosphorus oxychloride (5 ml) were used to synthesize and obtain a white solid V-36 (1.04 g, 55.55%).

MS (ESI): [M+H]⁺=469.

¹H-NMR (400 MHz, DMSO-d6) δppm: 3.05(t, 2H), 3.30(s, 3H), 3.45(t, 2H), 3.90(s, 2H), 4.06(s, NH, 1H), 5.15(s, 2H), 6.90(d, 1H), 7.01(m, 1H), 7.15(m, 1H), 7.23(s, 1H), 7.44(m, 1H), 7.58(m, 1 H), 7.72(m, 1 H), 7.96(s, 1 H), 8.24(s, 1 H).

### EXAMPLE 37

### 11-chloro-8-n-butyl-2,3-bis(2-methoxyethoxy)-8H-quinazolino[4,3-b]quinazoline (V-37)

4-chloro-6,7-bis(2-methoxyethoxy)quinazoline (3.12 g, 10 mmol) and 1-(2-amino-4-chlorophenyl)pentyl-1-alcohol (2.13 g, 10 mmol) were dissolved in isopropanol (30 ml) to form a solution, and concentrated sulfuric acid (0.20 ml) was added dropwise into the solution. The reaction was carried out according to General Method I for preparing the intermediate III to obtain a white solid intermediate M-32 (4.55 g, 86.67%).

M-32 (1.58 g, 3 mmol) was dissolved in CH₂Cl₂ (25 ml) to form a solution. In an ice water bath triethylamine (9 mmol) and methanesulfonyl chloride (15 mmol) were added sequentially into the solution. The reaction was carried out according to General Method I for preparing the product V to obtain a white solid V-37 (0.97 g, 68.65%).

MS (ESI): [M+H]⁺=472.

¹H-NMR(400 MHz, DMSO-d6) δppm: 1.25(t, J=7.8 Hz, 3H), 1.60-1.65(m, 4H), 2.12(q, J=7.6 Hz, 2H), 3.78(s, 6H), 3.96(m, 4H), 4.20(t, J=4.8 Hz, 2H), 4.3(t, J=4.4 Hz, 2H), 4.92(t, J=4.8 Hz, 1H), 6.85(s, 1H), 7.17(m, 1H), 7.40(m, 2H), 7.44(s, 1H), 8.25(s, 1H).

### EXAMPLE 38

### 11-chloro-2,3-bis(2-methoxyethoxy)-8-(2-methoxyethyl)-8H-quinazolino[4,3-b-]quinazoline (V-38)

4-chloro-6,7-bis(2-methoxyethoxy)quinazoline (3.12 g, 10 mmol) and 1-(2-amino-4-chlorophenyl)ethyl-1-alcohol (1.71 g, 10 mmol) were dissolved in isopropanol (30 ml) to form a solution, and concentrated hydrochloric acid (0.60 ml) was added dropwise to the solution. The reaction was carried out according to General Method I for preparing the intermediate III to obtain a white solid intermediate M-33 (3.25 g, 67.29%).

M-33 (1.45 g, 3 mmol) was dissolved in CH₂Cl₂ (25 ml) to form a solution, and in an ice water bath triethylamine (9 mmol) and methanesulfonyl chloride (15 mmol) were added dropwise sequentially to the solution. The reaction was carried out according to General Method I for preparing the product V to obtain a white solid V-38 (1.03 g, 72.59%).

MS (ESI): [M+H]+=474.

¹H-NMR(400 MHz, DMSO-d6) δppm: 3.05(q, 2H), 3.80(s, 9H), 3.92(t, J=4.4 Hz, 2H), 4.20(t, J=4.8 Hz, 4H), 4.35(t, J=4.4 Hz, 2H), 4.46(t, J=4.4 Hz, 2H), 5.21(t, J=4.8 Hz, 1H), 6.90(s, 1H), 7.06(m, 1H), 7.22(m, 1H), 7.40(m, 2H), 7.45(m, 1H), 8.33(s, 1H).

### EXAMPLE 39

### 2-chloro-11-cyano-8H-quinazolino[4,3-b]quinazoline (V-39)

According to the procedures of Example 1, 4,6-dichloro-quinazoline (1.98 g, 10 mmol) and 2-amino-4-cyano-benzyl alcohol (1.48 g, 10 mmol) were used to synthesize and obtain a white solid V-39 (1.54 g, the total yield of two steps was 52.74%).

MS (ESI): [M+H]⁺=293.

¹H-NMR (400 MHz, DMSO-d6) δppm: 4.40(s, 2H), 7.22(m, 1H), 7.40(m, 1H), 7.50(m, 1H), 7.52-7.55(m, 2H), 8.00(s, 1H), 8.22(s, 1H).

### EXAMPLE 40

### 11-chloro-2-nitro-8H-quinazolino[4,3-b]quinazoline (V-40)

According to the procedures of Example 1, 4-chloro-6-nitro-quinazoline (1.57 g, 10 mmol) and 2-amino-4-chloro-benzyl alcohol (2.09 g, 10 mmol) were used to synthesize and obtain a yellow solid V-40 (1.79 g, the total yield of the two steps was 57.37%).

MS (ESI): [M+H]⁺=313.

¹H-NMR (400 MHz, DMSO-d6) δppm: 4.15(s, 2H), 7.10(m, 1H), 7.26(s, 1H), 7.40(m, 1H), 7.52(m, 1H), 8.10(s, 1H), 8.22(m, 1H), 8.65(s, 1H).

### EXAMPLE 41

11-chloro-2-amino-8H-quinazolino[4,3-b]quinazoline (V-41) V-40 (0.32 g, 1 mmol) was dissolved in ethanol (10 ml), and stannous chloride dihydrate (0.67 g, 3 mmol) was added to form a yellow and clear solution. The reaction lasted for 7 h at room temperature. The reaction mixture was concentrated under vacuum to obtain a residue. 20 ml of water was added into the residue, and the pH was adjusted to 8 with 10% sodium hydroxide solution. A large quantity of solid then precipitated. The precipitate was filtered to obtain a crude product, which was then recrystallized by ethanol to obtain a white solid V-41 (2.20 g, 78.01%).

MS (ESI): [M+H]⁺=283.

¹H-NMR (400 MHz, DMSO-d6) δppm: 4.26(s, 2H), 6.25(s, NH₂, 2H), 6.95(m, 1H), 7.05(s, 1 H), 7.10(m, 1 H), 7.18 (m, 1H), 7.28(s, 1 H), 7.50(m, 1H), 8.15(s, 1H).

### EXAMPLE 42

### 11-chloro-2-methoxy-3-trifluoromethyl-8H-quinazolino[4,3-b]quinazoline (V-42)

4-chloro-6-methoxy-7-trifluoromethyl quinazoline (2.62, 10 mmol) and 2-amino-4-chlorobenzyl alcohol (1.57 g, 10 mol) were dissolved in isopropanol (30 ml) to form a solution, and concentrated hydrochloric acid (0.55 ml) was added dropwise to the solution. The reaction was carried out according to General Method I for preparing the intermediate III to obtain a white solid intermediate M-34 (3.33 g, 79.47%).

M-34 (1.26 g, 3 mmol) was dissolved in CH₂Cl₂ (25 ml) to form a solution, and in an ice water bath triethylamine (9 mmol) and methanesulfonyl chloride (15 mmol) were added dropwise sequentially to the solution. The reaction was carried out according to General Method I for preparing the product V to obtain a white solid V-42 (0.84 g, 76.71%).

MS (ESI): [M+H]⁺=366

¹H-NMR (400 MHz, CDCl₃) δppm: 3.80(s, 3H), 4.65(s, 2H), 7.10(d, J=8.0 Hz, 1H), 7.26(s, 1H), 7.40(m, 1 H), 7.58(s, 1H), 7.62(s, 1 H), 8.09(s, 1H).

### EXAMPLE 43

### 11-chloro-2-(3-fluoro-benzyloxy)3-methoxy-8H-quinazolino[4,3-b]quinazoline (V-43)

4-chloro-2- (3-fluoro-benzyloxy)-7-methoxy-quinazoline (3.18, 10 mmol) and 4-chloro-2-amino-benzyl alcohol (1.57 g, 10 mmol) were dissolved in isopropanol (30 ml) to form a solution, and phosphoric acid (0.20 ml) was added dropwise to the solution. The reaction was carried out according to General Method I for preparing the intermediate III to obtain a white solid intermediate M-35 (3.83 g, 80.63%).

M-35 (1.43 g, 3 mmol) was dissolved in CH₂Cl₂ (25 ml) to form a solution, and in an ice water bath triethylamine (9 mmol) and methanesulfonyl chloride (15 mmol) were added dropwise sequentially to the solution. The reaction was carried out according to General Method I for preparing the product V to obtain a white solid V-43 (0.72 g, 57.01%).

MS (ESI): [M+H]⁺=422.

¹H-NMR (400 MHz, DMSO-d6) δppm: 3.80(s, 3H), 4.25(s, 2H), 5.36(s, 2H), 6.85(s, 1 H), 6.97(s, 1H), 7.17-7.30(m, 4H), 7.40-7.42(m, 2H), 7.55(s, 1H), 8.18(s, 1H).

### EXAMPLE 44

### 9-bromo-3-methoxy-2-n-propyl-8H-quinazolino[4,3-b]quinazoline (V-44)

4-chloro-6-n-propyl-7-methoxy-quinazoline (2.36, 10 mmol) and 6-bromo-2-amino-benzyl alcohol (2.01 g, 10 mmol) were dissolved in isopropanol (35 ml), and heated to reflux for about 5 h. The reaction was carried out according to General Method I for preparing the intermediate III to obtain a white solid intermediate M-36 (3.65 g, 83.52%).

M-36 (1.75 g, 4 mmol) was dissolved in CH₂Cl₂ (25 ml) to form a solution, and in an ice water bath triethylamine (12 mmol) and methanesulfonyl chloride (20 mmol) were added dropwise sequentially to the solution. The reaction was carried out according to General Method I for preparing the product V to obtain a white solid V-44 (0.98 g, 63.97%).

MS (ESI): [M+H]⁺=384.

¹H-NMR (400 MHz, DMSO-d6) δppm: 1.52(t, J=4.4 Hz, 3H), 1.98(m, 2H), 3.05(t, J=4.8 Hz, 2H), 3.85(s, 3H), 4.46(s, 2H), 6.95(d, J=8.0 Hz, 1H), 7.08(m, 1H), 7.22(m, 1H), 7.40(m, 1H), 7.58(s, 1H), 8.34(s, 1H).

### EXAMPLE 45

### 9-bromo-8H-quinazolino[4,3-b]quinazoline-2-formic acid ethyl ester (V-45)

4-chloro-quinazoline-6-formic ether (2.36, 10 mmol) and 6-bromo-2-amino-benzyl alcohol (2.01 g, 10 mmol) were dissolved in isopropanol (30 ml) to form a solution, and concentrated hydrochloric acid (0.55 ml) was added dropwise to the solution. The reaction lasted for 7 h at room temperature, and the reaction mixture was filtered to obtain a white solid, which was dried under vacuum to obtain intermediate M-37 (3.32 g, 75.97%).

M-37 (1.31 g, 3 mmol) was dissolved in CH₂Cl₂ (25 ml) to form a solution, and in an ice water bath triethylamine (9 mmol) and methanesulfonyl chloride (12 mmol) were added dropwise sequentially to the solution. The reaction was carried out according to General Method I for preparing the product V to obtain a white solid V-45 (0.82 g, 71.18%).

MS (ESI): [M+H]⁺=384.

¹H-NMR (400 MHz, DMSO-d6) δppm: 2.10(t, J=8.0 Hz, 3H), 4.00(q, J=7.6 Hz, 2H), 5.15(s, 2H), 7.06(m, 1H), 7.15 (m, 1H), 7.48-7.52(m, 2H), 8.25(m, 1 H), 8.32(s, 1 H), 8.66(s, 1H).

### EXAMPLE 46

### (E)-(9-bromo-8H-quinazolino[4,3-b]quinazoline-2-yl)-4(dimethylamino)-2-butene amide hydrochloride (V-46)

According to the procedures of Example 5, N-(4-chloro-quinazoline-6-yl)-3-(dimethylamino) acrylamide (2.76 g, 10 mmol) and 4-bromo-2-amino-benzyl alcohol (2.20 g, 11 mmol) were used to obtain an off white solid compound V-46' (1.26 g, the total yield of two steps was 29.79%).

The solid compound V-46' obtained above was converted to V-46 according the procedures disclosed herein.

MS (ESI): [M+H]⁺=438.

¹H-NMR (400 MHz, DMSO-d6) δppm: 3.25(s, 6H), 3.42(d, J=4.6 Hz, 2H), 4.20(s, 2H), 5.35(d, J=16.2 Hz, 1H), 6.87(m, 1H), 7.12(m, 1H), 7.30(s, 1H), 7.51-7.55(m, 2H), 7.81(s, 1H), 8.23(s, 1H), 8.76(d, J=11.2 Hz, 1H), 10.24(s, 1 H, NH).

### EXAMPLE 47

### 9-bromo-8H-quinazolino[4,3-b]quinazoline-2-formic acid (V-47)

V-45 (0.25 g, 0.65 mmol) was dissolved in ethanol (15 ml) to form a solution, and 10% NaOH solution (0.15 ml) was added dropwise to the solution. The reaction lasted for 6 h at room temperature. Then the reaction mixture was dried under vacuum to obtain a residue. 10ml of water was added to the residue , and the pH was adjusted to 5 with 10% hydrochloric acid solution. A white solid precipitated. The precipitate was filtered to obtain a white solid V-47 (0.21 g,91.01%).

MS (ESI): [M+H]⁺=356

¹H-NMR (400 MHz, DMSO-d6) δppm: 4.20(s, 2H),6.88(m, 1H), 7.12(d, J=8 Hz, 1H), 7.22(m, 1H), 7.42(m, J=8.2 Hz, 1H), 7.65(m, 1H), 8.23(d, J=8.4 Hz, 1H), 8.56(s, 1H), 8.90(s, 1H), 11.21(s, 1H, OH).

### EXAMPLE 48

### 4-(2,3-dimethoxy-8H-quinazolino[4,3-b]quinazoline-9-yl)morpholine (V-48)

4-chloro-6,7-bis(2-methoxyethoxy)quinazoline (0.23 g, 1 mmol) and 1-(2-amino-6-morpholinyl-phenyl)-methanol (0.21 g, 1 mmol) were dissolved in CHCl₃ (50 ml) to form a solution, and concentrated hydrochloric acid (0.90 ml) was added dropwise to the solution. The reaction was carried out according to General Method I for preparing the intermediate III to obtain a white solid intermediate M-38 (0.32 g, 72.73%).

M-38 (0.43 g, 1 mmol) was dissolved in CH₂Cl₂ (7 ml) to form a solution, and in an ice water bath, diisopropylethylamine (3 mmol) and methanesulfonyl chloride (5 mmol) the added dropwise sequentially to the solution. The reaction was carried out according to General Method I for preparing the product V to obtain a white solid V-48 (0.12 g, 31.75%).

MS (ESI): [M+H]⁺=379

¹H-NMR (400 MHz, DMSO-d6) δppm: 3.25(m, 4H), 3.50(m, 4H), 3.85(s, 6H), 5.20(s, 2H), 6.50(d, J=8.0 Hz, 1H), 6.72(d, J=8.2Hz, 1 H), 6.95(s, 1 H), 7.12(m, 1H), 7.48(s, 1 H), 8.43(s, 1 H).

### EXAMPLE 49

### 11-chloro-2,3-dimethoxy-8-trifluoromethyl-8H-quinazolino[4,3-b]quinazoline (V-49)

Concentrated hydrochloric acid (0.55 ml) was added dropwise to 6,7-bis(2-methoxyethoxy)quinazoline (0.23 g, 1 mmol) and 1-(2-amino-4-Chlorophenyl)-2,2,2-trifluorobenzyl alcohol (0.23 g, 1 mmol). The reaction was carried out according to General Method I for preparing the intermediate III to obtain a white solid intermediate M-39 (0.35 g, 76.09%).

M-39 (0.46 g, 1 mmol) was dissolved in CH₂Cl₂ (7 ml) to form a solution, and in an ice water bath triethylamine (3 mmol) and methanesulfonyl chloride (5 mmol) were added dropwise sequentially to the solution. The reaction was carried out according to General Method I for preparing the product V to obtain a white solid V-49 (0.33 g, 83.54%).

MS (ESI): [M+H]⁺=396.

¹H-NMR (400 MHz, DMSO-d6) δppm: 3.85(s, 6H), 5.44(s, 2H), 6.87(s, 1H), 7.20(m, 1H), 7.33(s, 1H), 7.41(m, 1 H), 7.46(s, 1 H), 8.76(s, 1 H).

### EXAMPLE 50

### 4-chloro-6,7-bis(2-methoxyethoxy)quinazoline (compound I-1)

Ethyl 3,4-dihydroxybenzoate (36.4 g, 0.2 mol), potassium carbonate (82.8 g, 0.6 mol), and 2-methoxyethyl p-toluenesulfonate (72.0 g, 0.4 mol) were dissolved in acetonitrile (200 ml), heated and refluxed for 6 h. The solvent of the reaction mixture was evaporated under vacuum to obtain a residue. The residue was recrystallized by isopropanol, suction filtered, and dried under vacuum to obtain a white powder 3,4-bis (2-methoxyethoxy)ethyl benzoate (53.2 g, 89%).

3,4-bis(2-methoxyethoxy)benzoic acid ethyl ester (15.00 g, 0.05 mol) was added into acetic acid (50 ml) to form a mixture, and while the mixture was stirred in an ice water bath, 65%-68% nitric acid (13 ml) was added to the mixture. The mixture then was stirred for 24 h at room temperature. The reaction mixture was poured into ice water (500 ml), and extracted with ethyl acetate. The organic phase was combined, washed with saturated sodium bicarbonate solution three times, washed with saturated sodium chloride solution, and then dried by anhydrous sodium sulfate. The organic phase was then filtered to obtain a filtrate. The filtrate was concentrated to obtain a brown oily liquid 2-nitro-4,5-bis(2-methoxyethoxy)ethyl benzoate (14.10 g, 82.22%).

2-nitro-4,5-bis(2-methoxyethoxy)benzoic acid ethyl ester (34.3 g, 0.10 mol), ammonium formate (63 g, 1.00 mol), 5% Pd/C (5.00 g), and formamide (150 ml) were reacted for 7 h at 150□. The reaction mixture was cooled to room temperature, and a solid precipitated. The precipitate was filtered to obtain a white powder 6,7-bis-(2-methoxyethoxy)-4(3H)quinazolinone (22.1 g, 75%).

Thionyl chloride (14.9 g) was added dropwise slowly to a solution of 6,7-bis-(2-methoxyethoxy)-4 (3H) quinazolinone (14.7 g, 0.15 mol), N,N-dimethylformamide (1 ml), and dichloromethane (150 ml) under stirring at room temperature, heated and refluxed for 6 h. The reaction mixture was cooled to room temperature, and the pH of the reaction mixture was adjusted to 7-8 with a sodium hydroxide solution. The reaction mixture was then stirred for 30 min at room temperature, and set aside to until the organic and aqueous layers were separated. The organic layer was collected, and concentrated under vacuum to obtain a white solid 4-chloro-6,7-bis-(2-methoxyethoxy)-quinazoline (14.1 g, 89.5%), [M+H]⁺=313.

### EXAMPLE 51

### 2-amino-6-bromobenzyl alcohol (intermediate II-1)

2-nitro-6-bromo-toluene (32.20 g, 0.20 mol), N-bromosuccinimide (26.6 g, 0.20 mol), and benzoyl peroxide (0.40 g, 2 mmol) were dissolved in chlorobenzene (100 ml), and heated to reflux for about 20 h. The reaction mixture was filtered to obtain a filtrate. The filtrate was concentrated under vacuum to obtain a light brown oily product, which was recrystallized with ethanol to obtain a yellow solid 2-nitro-6-bromo benzyl bromide (47.31 g, 81%).

2-nitro-6-bromo benzyl bromide (14.70 g, 0.05 mol) and anhydrous sodium acetate(14.7 g,0.15 mol) were added into N,N-dimethylformamide (70 ml), heated and reacted for 1 h at 70□, and cooled the solution to room temperature. Water (150 ml) was added into the reaction mixture, extracted with ethyl acetate three times, washed with water three times, washed with saturated sodium chloride solution three times, dried by anhydrous sodium sulfate, and filtered. The filtrate was concentrated under vacuum to obtain a white solid 2-nitro-6-bromo phenyl acetic acid methyl ester (12.65 g, 92%).

2-nitro-6-bromo phenyl acetic acid methyl ester (2.73 g, 0.01 mol) was added into the water (100 ml), and 10% potassium hydroxide solution (20 ml) was added dropwise. The reaction mixture was heated to reflux for 2 h, cooled to room temperature, and extracted with ethyl acetate for three times. The organic phase was combined, washed with water and with saturated sodium chloride solution sequentially, dried by anhydrous sodium sulfate, and filtered. The filtrate was concentrated under vacuum to obtain a yellow solid 2-bromo-6-nitro-benzyl alcohol (1.91 g, 83%).

2-bromo-6-nitro benzyl alcohol (1.15 g, 5 mmol), sodium sulfide (0.78 g, 10 mmol), and sulfur (0.32 g, 10 mmol) were dissolved in ethanol (50 ml) and water (25 ml), heated to reflux for 2 h, and then cooled to room temperature. The ethanol was evaporated by concentration under vacuum to obtain a residue, and water (30 ml) was added into the residue, and extracted with ether three times. The organic phase was combined, washed with water and with saturated sodium chloride solution sequentially, dried by anhydrous sodium sulfate, and filtered. The filtrate was concentrated under vacuum to obtain a yellow solid 2-bromo-6-amino-benzyl alcohol (0.78 g, 78%), [M+H]⁺=202.

### EXAMPLE 52

### The inhibitory activities of EGFR tyrosine kinase in vitro

The inhibitory activity of compounds V-1 to V-36 on EGFR tyrosine kinase in vitro was tested according to the instruction of an EGFR inhibitor screening kit (Cat#PV3193, Invitrogen). The 200 µM and 40 µM solutions of compounds V-1 to V-36 were prepared, and the enzyme inhibition activities of the compounds V-1 to V-36 were tested at two concentrations respectively. Erlotinib hydrochloride was used as a positive control drug. The test results are listed in Table 1.

**Table 1 The inhibition ratio of the compound on EGFR(%)**

| Number | The inhibition ratio of the compound on EGFR(%) | |
|---|---|---|
| | 200µM | 40µM |
| V-1 | 91.5 | 57.6 |
| V-2 | 97.9 | 62.9 |
| V-3 | 81.6 | 49.5 |
| V-4 | 93.2 | 48.7 |
| V-5 | 74.6 | 25.2 |
| V-6 | 93.8 | 55.2 |
| V-7 | 80.5 | 36.2 |
| V-8 | 95.6 | 52.3 |
| V-9 | 60.3 | 13.0 |
| V-10 | 78.5 | 30.8 |
| V-11 | 85.6 | 56.4 |
| V-12 | 62.7 | 25.8 |
| V-13 | 88.9 | 45.7 |
| V-14 | 87.9 | 45.2 |
| V-15 | 94.0 | 60.7 |
| V-16 | 80.1 | 21.7 |
| V-17 | 70.6 | 25.4 |
| V-18 | 78.8 | 33.2 |
| V-19 | 95.7 | 53.4 |
| V-20 | 98.4 | 59.9 |
| V-22 | 95.6 | 63.2 |
| V-24 | 94.3 | 53.1 |
| V-28 | 98.7 | 64.4 |
| V-29 | 86.5 | 35.2 |
| V-30 | 82.1 | 27.8 |
| V-31 | 76.5 | 30.6 |
| V-33 | 97.9 | 64.3 |
| V-34 | 72.6 | 21.6 |
| V-35 | 75.8 | 13.3 |
| V-36 | 87.9 | 55.6 |
| Erlotinib hydrochloride | 81.7 | 37.9 |

### EXAMPLE 53

### The inhibitory activities of aurora kinase B in vitro

The inhibitory activities of compounds V-1 to V-36 on aurora kinase in vitro were tested according to the instructions of an aurora kinase screening kit (Cat #PV3174, Invitrogen). The 200 µM and 40 µM solutions of compounds V-1 to V-36 were prepared, and the enzyme inhibition activities of compounds V-1 to V-36 were tested at two concentrations respectively. Compound VX-680 (N-(4-(4-((5-methyl-1-H-pyrazole-3-yl)amino)-6-(4-methylpiperazine-1-yl)pyrimidine-2-yl)-thio)phenyl)cyclopropane carboxamide, an aurora kinase inhibitor) was used as a positive control drug. The testing results are listed in Table 2.

**Table 2 The inhibition ratio of the compound on Aurora B(%)**

| Number | The inhibition ratio of the compound on Aurora B(%) | |
|---|---|---|
| | 200µM | 40µM |
| V-1 | 88.6 | 47.6 |
| V-2 | 95.8 | 53.2 |
| V-3 | 83.6 | 39.5 |
| V-4 | 91.5 | 38.7 |
| V-5 | 74.6 | 35.1 |
| V-6 | 93.8 | 48.5 |
| V-7 | 91.7 | 23.2 |
| V-8 | 96.4 | 32.3 |
| V-9 | 60.5 | 17.6 |
| V-10 | 73.6 | 20.8 |
| V-11 | 96.7 | 53.5 |
| V-12 | 87.9 | 40.1 |
| V-13 | 95.8 | 55.2 |
| V-14 | 76.9 | 23.2 |
| V-15 | 92.5 | 58.7 |
| V-16 | 78.1 | 21.5 |
| V-17 | 84.3 | 26.4 |
| V-18 | 76.9 | 23.2 |
| V-19 | 95.7 | 52.4 |
| V-20 | 96.4 | 58.9 |
| V-22 | 95.6 | 57.2 |
| V-24 | 90.3 | 52.5 |
| V-28 | 78.7 | 45.2 |
| V-29 | 78.9 | 11.2 |
| V-30 | 79.6 | 46.3 |
| V-31 | 66.5 | 10.6 |
| V-33 | 93.7 | 54.6 |
| V-34 | 92.6 | 17.8 |
| V-35 | 79.8 | 3.3 |
| V-36 | 97.9 | 52.6 |
| VX-680 | 81.6 | 35.2 |

The structure of VX-680 is shown below:

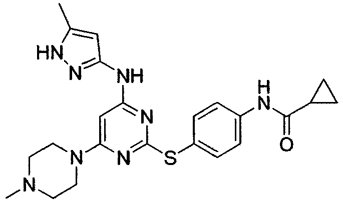

### EXAMPLE 54

### The inhibitory activities of the compounds on tumor cells in vitro

The inhibition ratios of the compounds on Hut78 T-Lymphocytic Leukemia cells, Jurkat E6-1 human T-cell lymphoma, PANC-1 human pancreatic cancer cells, A549 human lung cancer cells, K562 human chronicity marrow leukemic cells, Hep3B2.1-7 human hepatoma carcinoma cells, MDA-MB-435s human breast carcinoma cells, Colo320 human rectal cancer cells, PC-3 human carcinoma of prostate, and HepG2 human hepatoma carcinoma cells were determined using a CCK-8(Dojindo Molecular Technologies, Inc.) kit. The concentrations of the compounds tested were 100 µM and 10µM. Erlotinib hydrochloride and gefitinib were employed as positive control drugs. The test results are listed in Table 3 and Table 4.

Table 3 Antiproliferative effect of compounds on Jurkat E6-1, Hut78, Colo320, K562, and 435s cells

| Number | E6-1 | | Hut-78 | | Colo320 | | K562 | | 435s | |
|---|---|---|---|---|---|---|---|---|---|---|
| | 100 µM | 10 µM | 100 µM | 10 µM | 100 µM | 10 µM | 100 µM | 10 µM | 100 µM | 10 µM |
| V-1 | 81.67 | 55.01 | 64.54 | 16.15 | 80.98 | 55.14 | 87.03 | 40.66 | 91.20 | 60.85 |
| V-2 | 83.12 | 56.24 | 73.79 | 28.95 | 89.60 | 56.99 | 91.10 | 65.44 | 82.59 | 59.83 |
| V-3 | 78.02 | 42.02 | 84.14 | 44.14 | 78.98 | 34.77 | 88.79 | 43.14 | 84.12 | 3.38 |
| V-4 | 19.79 | 6.85 | 85.01 | 44.10 | 83.38 | 48.91 | 87.58 | 31.44 | 92.57 | 31.31 |
| V-5 | 78.86 | 34.78 | 63.55 | 43.77 | 84.94 | 51.70 | 78.27 | 23.63 | 87.20 | 48.94 |
| V-6 | 95.35 | 61.09 | 91.87 | 68.97 | 99.15 | 73.46 | 82.83 | 64.13 | 86.97 | 50.42 |
| V-7 | 75.38 | 34.56 | 62.41 | 32.06 | 81.38 | 42.11 | 61.68 | 24.29 | 81.39 | 37.96 |
| V-8 | 85.41 | 44.00 | 75.93 | 35.58 | 94.85 | 64.49 | 92.19 | 52.43 | 93.71 | 55.02 |
| V-9 | 65.43 | 20.40 | 70.55 | 19.24 | 73.29 | 46.27 | 79.88 | 34.63 | 80.74 | 54.85 |
| V-10 | 84.15 | 51.83 | 72.25 | 41.08 | 72.81 | 10.76 | 84.99 | 47.54 | 65.10 | 29.59 |
| V-11 | 90.80 | 41.62 | 72.78 | 30.99 | 96.30 | 41.56 | 85.98 | 50.45 | 89.51 | 41.01 |
| V-13 | 97.97 | 52.59 | 101.39 | 66.16 | 483.64 | 59.70 | 98.46 | 66.15 | 67.92 | 26.76 |
| V-14 | 97.71 | 26.85 | 98.69 | 21.28 | 88.00 | 17.56 | 100.49 | 36.61 | 94.77 | 46.62 |
| V-15 | 85.81 | 69.49 | 93.20 | 60.98 | 96.46 | 58.29 | 91.51 | 45.20 | 92.03 | 72.61 |
| V-16 | 97.30 | 56.63 | 99.57 | 44.27 | 99.40 | 52.71 | 93.75 | 53.63 | 66.20 | 48.94 |
| V-17 | 79.56 | 33.69 | 45.28 | 35.91 | 26.05 | 9.66 | 25.20 | 19.15 | 95.49 | 37.48 |
| V-18 | 84.05 | 44.09 | 65.15 | 19.72 | 75.06 | 38.13 | 59.03 | 10.45 | 72.26 | 36.58 |
| V-19 | 91.12 | 55.24 | 63.79 | 32.95 | 89.60 | 36.99 | 81.10 | 45.44 | 91.59 | 55.83 |
| V-20 | 95.63 | 57.91 | 82.14 | 52.33 | 92.27 | 66.86 | 97.44 | 40.66 | 93.88 | 65.72 |
| V-21 | 90.31 | 43.50 | 52.69 | 10.33 | 87.01 | 31.75 | 81.57 | 26.92 | 74.86 | 66.97 |
| V-22 | 98.02 | 67.02 | 94.64 | 54.14 | 98.98 | 43.77 | 88.79 | 23.14 | 94.12 | 63.38 |
| V-23 | 77.52 | 56.28 | 99.80 | 42.03 | 99.54 | 52.32 | 73.37 | 58.67 | 52.17 | 20.68 |
| V-24 | 87.30 | 46.63 | 99.57 | 44.27 | 99.40 | 52.71 | 93.75 | 53.63 | 66.20 | 48.94 |
| V-25 | 81.35 | 31.09 | 21.87 | 18.97 | 79.15 | 33.46 | 82.83 | 44.13 | 76.97 | 20.42 |
| V-26 | 85.38 | 44.56 | 72.41 | 32.06 | 81.38 | 32.11 | 11.68 | 4.29 | 71.39 | 37.96 |
| V-27 | 75.41 | 24.00 | 95.93 | 65.58 | 84.85 | 34.49 | 62.19 | 22.43 | 79.71 | 12.02 |
| V-28 | 95.43 | 40.40 | 90.55 | 59.24 | 63.29 | 16.27 | 99.88 | 64.63 | 90.74 | 54.85 |
| V-31 | 84.15 | 21.83 | 72.25 | 41.08 | 92.81 | 30.76 | 84.99 | 37.54 | 85.10 | 50.59 |
| V-33 | 90.80 | 41.62 | 92.78 | 60.99 | 96.30 | 61.56 | 85.98 | 40.45 | 99.51 | 62.74 |
| Erlotinib Hydrochloride | 75.63 | 47.91 | 62.14 | 42.33 | 62.27 | 56.86 | 67.44 | 40.66 | 83.88 | 35.72 |
| Gefitinib | 70.12 | 40.59 | 70.36 | 35.41 | 65.42 | 44.31 | 78.52 | 10.02 | 80.01 | 31.13 |

**Table 4 Antiproliferative effect of compounds on Hep3B, A549, PANC-1, PC-3 cells**

| Number | Hep3B | | A549 | | PANC-1 | | PC-3 | |
|---|---|---|---|---|---|---|---|---|
| | 100 µM | 10 µM | 100 µM | 10 µM | 100 µM | 10 µM | 100 µM | 10 µM |
| V-1 | 89.26 | 50.37 | 81.43 | 40.52 | 78.10 | 35.67 | 62.37 | 51.62 |
| V-2 | 90.00 | 53.45 | 90.69 | 68.62 | 95.91 | 59.74 | 92.73 | 72.25 |
| V-3 | 80.66 | 42.32 | 76.22 | 44.59 | 75.40 | 30.87 | 86.95 | 33.98 |
| V-4 | 79.00 | 33.45 | 78.69 | 48.62 | 85.91 | 39.74 | 62.73 | 22.25 |
| V-5 | 54.40 | 22.27 | 92.45 | 59.76 | 80.30 | 17.08 | 63.57 | 32.50 |
| V-6 | 84.67 | 51.34 | 97.30 | 63.01 | 98.57 | 64.17 | 84.37 | 53.36 |
| V-7 | 89.48 | 40.43 | 83.61 | 27.69 | 86.58 | 40.71 | 90.30 | 15.86 |
| V-8 | 87.27 | 52.44 | 79.97 | 52.80 | 89.78 | 41.46 | 93.96 | 45.18 |
| V-9 | 82.77 | 40.14 | 87.51 | 33.72 | 55.56 | 11.72 | 78.03 | 16.37 |
| V-10 | 72.21 | 31.90 | 82.78 | 28.77 | 96.90 | 36.52 | 91.73 | 9.39 |
| V-11 | 82.82 | 57.10 | 99.18 | 63.07 | 91.27 | 57.93 | 89.67 | 55.07 |
| V-13 | 102.34 | 64.71 | 91.08 | 43.04 | 87.24 | 45.92 | 87.74 | 61.35 |
| V-14 | 100.18 | 45.14 | 97.84 | 31.10 | 99.90 | 38.29 | 97.08 | 38.52 |
| V-15 | 97.36 | 52.36 | 90.76 | 75.64 | 94.14 | 61.77 | 81.32 | 42.36 |
| V-16 | 74.56 | 30.33 | 70.22 | 50.69 | 99.90 | 22.38 | 98.89 | 53.50 |
| V-17 | 85.22 | 58.35 | 87.14 | 49.89 | 56.76 | 19.56 | 91.67 | 28.12 |
| V-18 | 77.45 | 40.15 | 78.56 | 37.79 | 84.38 | 30.01 | 76.16 | 36.99 |
| V-19 | 85.00 | 53.45 | 90.69 | 48.62 | 85.91 | 49.74 | 92.73 | 62.25 |
| V-20 | 92.23 | 60.73 | 90.70 | 55.03 | 88.25 | 54.79 | 75.15 | 46.75 |
| V-21 | 86.31 | 21.82 | 78.04 | 45.28 | 89.44 | 52.67 | 62.58 | 27.61 |
| V-22 | 90.66 | 62.32 | 96.22 | 54.59 | 95.40 | 60.87 | 96.95 | 63.98 |
| V-23 | 48.66 | 27.12 | 22.52 | 10.43 | 98.80 | 21.54 | 97.60 | 30.69 |
| V-24 | 94.56 | 60.33 | 98.22 | 50.69 | 99.90 | 42.38 | 98.89 | 53.50 |
| V-25 | 64.67 | 21.34 | 79.30 | 47.01 | 88.57 | 44.17 | 64.37 | 53.36 |
| V-26 | 89.48 | 28.43 | 93.61 | 27.69 | 76.58 | 40.71 | 30.30 | 5.86 |
| V-27 | 67.27 | 2.44 | 79.97 | 39.80 | 99.78 | 41.76 | 83.96 | 25.18 |
| V-28 | 92.77 | 40.14 | 97.51 | 53.72 | 95.56 | 51.72 | 88.03 | 56.37 |
| V-31 | 62.21 | 31.90 | 32.78 | 28.77 | 96.40 | 46.52 | 81.73 | 29.39 |
| V-33 | 86.48 | 51.30 | 86.06 | 48.09 | 87.99 | 50.30 | 78.15 | 16.47 |
| Erlotinib Hydrochloride | 52.23 | 30.73 | 85.70 | 34.03 | 78.25 | 14.79 | 54.15 | 24.75 |
| Gefitinib | 70.31 | 25.52 | 76.82 | 30.01 | 58.83 | 21.37 | 55.46 | 26.72 |

### EXAMPLE 55

### The inhibitory activities of the compounds on tumor cells (IC₅₀) in vitro

The IC₅₀ (IC₅₀, unit: µg/ml) values of the compounds on Hut78 T-Lymphocytic Leukemia cells, Jurkat E6-1 human T-cell lymphoma, PANC-1 human pancreatic cancer cells, A549 human lung cancer cells, K562 human chronicity marrow leukemic cells, Hep3B2.1-7 human hepatoma carcinoma cells, MDA-MB-435s human breast carcinoma cells, Colo320 human rectal cancer cells, PC-3 human carcinoma of prostate, and HepG2 human hepatoma carcinoma cells were determined using a CCK-8 kit. Erlotinib hydrochloride was used as the positive control drug. The test results are listed in Table 5.

**Table 5 IC₅₀ of compounds on E6-1, Hut-78, Colo320, K562, 435s, Hep3B, A549, PANC-1, and PC-3 cells**

| Number | E6-1 | Hut-78 | Colo320 | K562 | 435s | Hep3B | A549 | PANC1 | PC-3 |
|---|---|---|---|---|---|---|---|---|---|
| V-2 | 15.31 | 61.15 | 32.31 | 33.96 | 0.42 | 9.83 | 0.67 | 0.11 | 4.78 |
| V-6 | 30.46 | 58.75 | 29.78 | 46.35 | 33.46 | 15.60 | 3.68 | 16.78 | 32.99 |
| V-8 | 8.11 | 53.12 | 29.76 | 63.12 | 27.63 | 33.15 | 30.01 | 21.17 | 9.90 |
| V-11 | 39.88 | 26.78 | 34.56 | 40.59 | 6.79 | 26.11 | 4.64 | 6.72 | 36.79 |
| V-13 | 3.89 | 0.62 | 9.88 | 10.99 | 31.43 | 0.32 | 10.72 | 14.88 | 9.78 |
| V-15 | 5.72 | 14.33 | 55.62 | 1.50 | 21.00 | 6.91 | 1.01 | 8.32 | 24.62 |
| V-19 | 12.46 | 47.38 | 10.03 | 31.11 | 36.54 | 9.32 | 50.03 | 27.34 | 15.33 |
| V-20 | 0.05 | 55.31 | 13.14 | 10.10 | 16.78 | 71.43 | 12.13 | 3.72 | 22.66 |
| V-22 | 13.33 | 11.36 | 4.67 | 19.64 | 6.18 | 14.41 | 9.87 | 84.31 | 7.69 |
| V-24 | 9.86 | 2.14 | 4.76 | 10.32 | 53.36 | 43.12 | 23.27 | 1.94 | 3.41 |
| V-28 | 10.01 | 16.71 | 59.68 | 7.84 | 16.31 | 36.75 | 6.42 | 18.77 | 31.43 |
| V-33 | 15.39 | 13.42 | 7.83 | 62.56 | 10.30 | 22.70 | 0.05 | 1.97 | 40.66 |
| Erlotinib Hydrochloride | 35.64 | 43.21 | 40.39 | 39.88 | 13.12 | 62.31 | 19.33 | 32.54 | 54.21 |

### EXAMPLE 56

### Acute Toxicity Study

The acute toxicity experiments were carried out according to the methods disclosed in the book of Modern Pharmacological Methods, edited chiefly by Zhang Jun-Tian. Through preliminary screening using the Bliss statistical method, the LD₅₀ of compounds V-2, V-15, V-20, V-22, and V-33 were determined to be 2.05 g/kg, 2.97 g/kg, 0.75 g/kg, 1.34 g/kg, and 2.84 g/kg respectively.

### EXAMPLE 57

Tablets of the compounds disclosed herein can be made according to the following recipe:

| | |
|---|---|
| Active Pharmaceutical Ingredient (At least one of compound V1-V49) | 10mg |
| Sucrose | 150mg |
| Corn Starch | 38mg |
| Calcium Striethylaminerate | 2mg |

To a mixture of active pharmaceutical ingredient, sucrose and corn starch is added an appropriate amount of water. The mixture is stirred until it is uniform. The mixture is then dried, crushed, and sieved. Calcium striethylaminerate is then added to the mixture and mixed well, and then the resulting mixture is pressed into tablets. Each tablet weighs 200mg, which contains 10mg pharmaceutically active ingredient.

### EXAMPLE 58

A formulation for injection can be made according to the following recipe:

| | |
|---|---|
| Active Pharmaceutical Ingredient (At least one of compound V1-V49) | 10mg |
| Water for Injection | 2mg |

The active pharmaceutical ingredient can be dissolved in water for injection, mixed, and filtered. The solution obtained can be subpackaged into sterile ampoule bottles under sterile conditions. The solution in one bottle can be 10 mg, which contains 2mg of the active pharmaceutical ingredient disclosed herein.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. An active pharmaceutical ingredient of formula V, 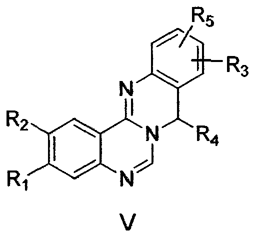 or a Pharmaceutical acceptable salt thereof, or a hydrate of a pharmaceutically acceptable salt thereof,
wherein, R₁ or R₂ is independently chosen from hydrogen, halogen, amino, nitro, hydroxamino, carboxyl, C₁-C₄ alkoxy carbonyl, C₁-C₄ alkyl, C₁-C₄ acylamino, (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₃ or R₅ is independently chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino, benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic, substituted benzyloxy;
R₄ is chosen from hydrogen, trifluoromethyl, C₁-C₄ alkyl and C₁-C₄ alkoxy;
R₁, R₂, R₃, R₄ and R₅ are not simultaneously hydrogen.

2. An active pharmaceutical ingredient according to claim 1, wherein the substituted phenyl has 1 to 4 substituents independently chosen from halogen, hydroxyl, nitro, cyano, C₁-C₄ alkoxy, C₁-C₄ alkyl, and amino.

3. An active pharmaceutical ingredient according to claim 1, wherein the heterocyclic is an saturated or unsaturated, five or six membered ring containing at least one heteroatom, the heteroatom herein is chosen from N, O and S.

4. An active pharmaceutical ingredient according to claim 2, wherein the heterocyclic is an saturated or unsaturated, five or six membered ring containing at least one heteroatom, the heteroatom herein is chosen from N, O and S.

5. An active pharmaceutical ingredient according to any of claims 1 to 4, wherein the heterocyclic herein is 5-((2-(methylsulfonyl)ethylamino)methyl)furyl.

6. An active pharmaceutical ingredient according to any of claims 1 to 4, wherein the alkoxy is chosen from C₁-C₄ alkoxy, substituted benzyloxy, pyrrolidine-1-yl-(C₂-C₄)alkoxy, morpholino-1-yl-(C₂-C₄)alkoxy, piperazin-1-yl-(C₂-C₄)alkoxy, N-methyl piperazine-1-yl-(C₂-C₄) alkoxy and piperidine-1-yl-(C₂-C₄)alkoxy.

7. An active pharmaceutical ingredient according to claim 5, wherein the alkoxy is chosen from C₁-C₄ alkoxy, substituted benzyloxy, pyrrolidine-1-yl-(C₂-C₄)alkoxy, morpholino-1-yl-(C₂-C₄)alkoxy, piperazin-1-yl-(C₂-C₄)alkoxy, N-methyl piperazine-1-yl-(C₂-C₄) alkoxy and piperidine-1-yl-(C₂-C₄)alkoxy.

8. An active pharmaceutical ingredient according to any of claims 1, 2, 3, 4 and 7, wherein the halogen is chosen from fluorine, chlorine, bromine, and iodine.

9. An active pharmaceutical ingredient according to claim 5, wherein the halogen is chosen from fluorine, chlorine, bromine, and iodine.

10. An active pharmaceutical ingredient according to claim 6, wherein the halogen is chosen from fluorine, chlorine, bromine, and iodine.

11. An active pharmaceutical ingredient according to any of claims 1, 2, 3, 4, 7, 9 and 10, wherein the pharmaceutically acceptable salts are chosen from hydrochloride, hydrobromide, sulfate, acetate, lactate, tartrate, tannate, citrate, triflouroacetate, malate, maleate, succinate, p-toluenesulfonate, and mesylate.

12. An active pharmaceutical ingredient according to claim 5, wherein the pharmaceutically acceptable salts are chosen from hydrochloride, hydrobromide, sulfate, acetate, lactate, tartrate, tannate, citrate, triflouroacetate, malate, maleate, succinate, p-toluenesulfonate, and mesylate.

13. An active pharmaceutical ingredient according to claim 6, wherein the pharmaceutically acceptable salts are chosen from hydrochloride, hydrobromide, sulfate, acetate, lactate, tartrate, tannate, citrate, triflouroacetate, malate, maleate, succinate, p-toluenesulfonate, and mesylate.

14. An active pharmaceutical ingredient according to claim 8, wherein the pharmaceutically acceptable salts are chosen from hydrochloride, hydrobromide, sulfate, acetate, lactate, tartrate, tannate, citrate, triflouroacetate, malate, maleate, succinate, p-toluenesulfonate, and mesylate.

15. An active pharmaceutical ingredient according to any of claims 1, 2, 3, 4, 7, 9, 10, 12, 13 and 14, wherein the C₁-C₄ alkoxy is chosen from methoxyl, ethoxyl, n-propoxyl, iso-propoxyl, n-butoxyl, iso-butoxyl, methoxyethoxyl and ethoxymethoxyl.

16. An active pharmaceutical ingredient according to claim 5, wherein the C₁-C₄ alkoxy is chosen from methoxyl, ethoxyl, n-propoxyl, iso-propoxyl, n-butoxyl, iso-butoxyl, methoxyethoxyl and ethoxymethoxyl.

17. An active pharmaceutical ingredient according to claim 6, wherein the C₁-C₄ alkoxy is chosen from methoxyl, ethoxyl, n-propoxyl, iso-propoxyl, n-butoxyl, iso-butoxyl, methoxyethoxyl, ethoxymethoxyl.

18. An active pharmaceutical ingredient according to claim 8, wherein the C₁-C₄ alkoxy is chosen from methoxyl, ethoxyl, n-propoxyl, iso-propoxyl, n-butoxyl, iso-butoxyl, methoxyethoxyl, ethoxymethoxyl.

19. An active pharmaceutical ingredient according to claim 11, wherein the C₁-C₄ alkoxy is chosen from methoxyl, ethoxyl, n-propoxyl, iso-propoxyl, n-butoxyl, iso-butoxyl, methoxyethoxyl, ethoxymethoxyl.

20. An active pharmaceutical ingredient according to any of claims 1, 2, 3, 4, 7, 9, 10, 12, 13, 14, 16, 17, 18 and 19, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl,ethoxymethyl, ethoxyethyl, n-propoxymethyl, iso-propoxymethyl, the C₁-C₄ acylamino is chosen from acetamido, n-propionamido, n-butanamido, iso-butanamido.

21. An active pharmaceutical ingredient according to claim 5, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl, ethoxymethyl, ethoxyethyl, n-propoxymethyl, iso-propoxymethyl, the C₁-C₄ acylamino is chosen from acetamido, n-propionamido, n-butanamido, iso-butanamido.

22. An active pharmaceutical ingredient according to claim 6, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl, ethoxymethyl, ethoxyethyl, n-propoxymethyl, iso-propoxymethyl, the C₁-C₄ acylamino is are chosen from acetamido, n-propionamido, n-butanamido, iso-butanamido.

23. An active pharmaceutical ingredient according to claim 8, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl, ethoxymethyl, ethoxyethyl, n-propoxymethyl, iso-propoxymethyl, the C₁-C₄ acylamino is are chosen from acetamido, n-propionamido, n-butanamido, iso-butanamido.

24. An active pharmaceutical ingredient according to claim 11, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl, ethoxymethyl, ethoxyethyl, n-propoxymethyl, iso-propoxymethyl, the C₁-C₄ acylamino is chosen from acetamido,n-propionamido, n-butanamido, iso-butanamido.

25. An active pharmaceutical ingredient according to claim 15, wherein the C₁-C₄ alkyl is chosen from methyl, ethyl, n-propyl, iso-propyl, cyclopropyl, n-butyl, iso-butyl, sec-butyl, tert-butyl, cyclobutyl, methoxyethyl, methoxy n-propyl, methoxy iso-propyl,ethoxymethyl, ethoxyethyl, n-propoxymethyl, iso-propoxymethyl, the C₁-C₄ acylamino is chosen from acetamido,n-propionamido, n-butanamido, iso-butanamido.

26. An active pharmaceutical ingredient according to claim 1, wherein R₁ is chosen from hydrogen, halogen, amino, nitro, hydroxamino, carboxyl, C₁-C₄ alkoxy carbonyl, C₁-C₄ alkyl, C₁-C₄ acylamino, (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₂ is chosen from hydrogen, halogen, amino, nitro, hydroxamino, carboxyl, C₁-C₄ alkoxy carbonyl, C₁-C₄ alkyl, C₁-C₄ acylamino, (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic;
R₃ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, substituted C₁-C₄ amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic and substituted benzyloxy;
R₄ is chosen from hydrogen, trifluoromethyl, C₁-C₄ alkyl and C₁-C₄ alkoxy;
R₅ is chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino, benzamido, substituted C₁-C₄ amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic and substituted benzyloxy;
R₁, R₂, R₃, R₄ and R₅ are not simultaneously hydrogen.

27. An active pharmaceutical ingredient according to claim 1, wherein R₁ is chosen from hydrogen, halogen, amino, nitro, hydroxamino, carboxyl, ethoxy methylcarbonyl, n-propyl, (E)-4-(dimethylamino)-2-butene acylamino, methoxyl, 2-methoxy ethoxy, trifluoromethyl, 3-fluoro-benzyloxy, pyrrolidine-1-yl-2-propoxy, morpholino-1-yl-2-propoxy, piperazin-1-yl-2-propoxy, N-methyl piperazine-1-yl-2-propoxy, piperidine-1-yl-2-propoxy and 5-((2-(methylsulfonyl)ethylamino)methyl)furyl;
R₂ is chosen from hydrogen, halogen, amino, nitro, hydroxamino, carboxyl, ethoxy methylcarbonyl, n-propyl, (E)-4-(dimethylamino)-2-butene acylamino, methoxyl, 2-methoxy ethoxy, trifluoromethyl, 3-fluoro-benzyloxy, pyrrolidine-1-yl-2-propoxy, morpholino-1-yl-2-propoxy, piperazin-1-yl-2-propoxy, N-methyl piperazine-1-yl-2-propoxy, piperidine-1-yl-2-propoxy and 5-((2-(methylsulfonyl)ethylamino)methyl)furyl;
R₃ is chosen from hydrogen, halogen, trifluoromethyl, cyano, ethynyl, nitro, amino, hydroxyl, hydroxymethyl, methanol methanesulfonic acid ester, methoxyl, acetamido, benzamido, 3-fluoro-benzyloxy and morpholino;
R₄ is chosen from hydrogen, methyl, trifluoromethyl, n-butyl, methoxyethyl;
R₅ is chosen from hydrogen, halogen, trifluoromethyl, cyano, ethynyl, nitro, amino, hydroxyl, hydroxymethyl, methanol methanesulfonic acid ester, methoxyl, acetamido, benzamido, 3-fluoro-benzyloxy and morpholino;
R₁, R₂, R₃, R₄, R₅ are not simultaneously hydrogen.

28. An active pharmaceutical ingredient according to claim 1 chosen from:
9-chloro-2,3-bis(2-methoxyethoxy)-8H-quinazolino[4,3-b]quinazoline hydrochloride monohydrate,
9-chloro-2,3-bis(2-methoxyethoxy)-8H-quinazolino[4,3-b]quinazoline,
9-bromo-2,3-bis(2-methoxyethoxy)-8H-quinazolino[4,3-b]quinazoline hydrochloride hemihydrate,
9-bromo-2,3-bis(2-methoxyethoxy)-8H-quinazolino[4,3-b]quinazoline,
9-fluoro-2,3-bis(2-methoxyethoxy)-8H-quinazolino[4,3-b]quinazoline mesylate,
9-fluoro-2,3-bis(2-methoxyethoxy)-8H-quinazolino[4,3-b]quinazoline,
9-ethynyl-2,3-bis(2-methoxyethoxy)-8H-quinazolino[4,3-b]quinazoline hydrochloride trihydrate,
9-ethynyl-2,3-bis(2-methoxyethoxy)-8H-quinazolino[4,3-b]quinazoline,
9-nitro-2,3-bis(2-methoxyethoxy)-8H-quinazolino[4,3-b]quinazoline,
9-amino-2,3-bis(2-methoxyethoxy)-8H-quinazolino[4,3-b]quinazoline hydrobromide trihydrate,
9-amino-2,3-bis(2-methoxyethoxy)-8H-quinazolino[4,3-b]quinazoline,
9-cyano-2,3-bis(2-methoxyethoxy)-8H-quinazolino[4,3-b]quinazoline,
9-hydroxy-2,3-bis(2-methoxyethoxy)-8H-quinazolino[4,3-b]quinazoline,
9-trifluoromethyl-2,3-bis(2-methoxyethoxy)-8H-quinazolino[4,3-b]quinazoline,
9-methoxy-2,3-bis(2-methoxyethoxy)-8H-quinazolino[4,3-b]quinazoline,
N-{2,3-bis(2-methoxyethoxy)-8H-quinazolino[4,3-b]quinazoline}acetamide,
N-{2,3-bis(2-methoxyethoxy)-8H-quinazolino[4,3-b]quinazoline}benzamide, 9-chloro-10-fluoro-2,3-bis(2-methoxyethoxy)-8H-quinazolino[4,3-b]quinazoline,
9-chloro-10-(3-ffuoro-benzyloxy)-2,3-bis(2-methoxyethoxy)-8H-quinazolino[4,3-b]quin azoline,
11-chloro-2,3-bis(2-methoxyethoxy)-8H-quinazolino[4,3-b]quinazoline,
11-fluoro-2,3-bis(2-methoxyethoxy)-8H-quinazolino[4,3-b]quinazoline,
11-cyano-2,3-bis(2-methoxyethoxy)-8H-quinazolino[4,3-b]quinazoline,
11-hydroxy-2,3-bis(2-methoxyethoxy)-8H-quinazolino[4,3-b]quinazoline,
11-ethynyl-2,3-bis(2-methoxyethoxy)-8H-quinazolino[4,3-b]quinazoline,
mesylate-{9-nitro-2,3-bis(2-methoxyethoxy)-8H-quinazolino[4,3-b]quinazoline-11-met hanol}ester hydrochloride,
mesylate-{9-nitro-2,3-bis(2-methoxyethoxy)-8H-quinazolino[4,3-b]quinazoline-11-met hanol}ester,
9-amino-11-hydroxymethyl-2,3-bis(2-methoxyethoxy)-8H-quinazolino[4,3-b]quinazoli ne,
11-chloro-8-methyl-2,3-bis(2-methoxyethoxy)-8H-quinazolino[4,3-b]quinazoline malate,
11-chloro-8-methyl-2,3-bis(2-methoxyethoxy)-8H-quinazolino[4,3-b]quinazoline,
4-(3-((9-chloro-10-fluoro-3-methoxy-8H-quinazolino[4,3-b]quinazoline-2-yl)oxy)propyl )morpholine di-p-toluenesulfonate trihydrate,
4-(3-((9-chloro-10-fluoro-3-methoxy-8H-quinazolino[4,3-b]quinazoline-2-yl)oxy)propyl )morpholine,
9-chloro-3-methoxy-2-(3-(pyrrolidine-1-yl)propoxy)-8H-quinazolino[4,3-b]quinazoline,
9-fluoro-3-methoxy-2-(3-(piperazinyl-1-yl)propoxy)-8H-quinazolino[4,3-b]quinazoline,
4-(3-((9-ethynyl-3-methoxy-8H-quinazolino[4,3-b]quinazoline-2-yl)oxy)propyl)morphol ine,
3-methoxy-2-(3-morpholinyl propoxy)-8H-quinazolino[4,3-b]quinazoline-9-nitrile,
4-(3-((11-chloro-3-methoxy-8H-quinazolino[4,3-b]quinazoline-2-yl)oxy)propyl)morphol ine,
mesylate-{3-(((9-nitro-8H-quinazolino[4,3-b]quinazoline-2-yl)oxy)propyl)morpholine-1 1-methanol}ester,
9-chloro-10-fluoro-2,3-dimethoxy-8H-quinazolino[4,3-b]quinazoline, 9-ethynyl-2,3-dimethoxy-8H-quinazolino[4,3-b]quinazoline,
9-cyano-2,3-dimethoxy-8H-quinazolino[4,3-b]quinazoline,
N-((5-(9-chloro-10-(3-fluorobenzyloxy)-8H-quinazolino[4,3-b]quinazoline-2-yl)furan-2-yl)methyl)-2-(methylsulfonyl)ethylamine,
N-((5-(9-chloro-10-fluoro-8H-quinazolino[4,3-b]quinazoline-2-yl)furan-2-yl)methyl)-2-( methylsulfonyl)ethylamine,
N-((5-(9-ethynyl-8H-quinazolino[4,3-b]quinazoline-2-yl)furan-2-yl)methyl)-2-(methylsu lfonyl)ethylamine,
N-((5-(11-chloro-8H-quinazolino[4,3-b]quinazoline-2-yl)furan-2-yl)methyl)-2-(methylsu lfonyl)ethylamine,
11-chloro-8-n-butyl-2,3-bis(2-methoxyethoxy)-8H-quinazolino[4,3-b]quinazoline,
11-chloro-2,3-bis(2-methoxyethoxy)-8-(2-methoxyethyl)-8H-quinazolino[4,3-b]quinaz oline,
2-chloro-11-cyano-8H-quinazolino[4,3-b]quinazoline,
11-chloro-2-nitro-8H-quinazolino[4,3-b]quinazoline,
11-chloro-2-amino-8H-quinazolino[4,3-b]quinazoline,
11-chloro-2-methoxy-3-trifluoromethyl-8H-quinazolino[4,3-b]quinazoline,
11-chloro-2-(3-fluoro-benzyloxy)3-methoxy-8H-quinazolino[4,3-b]quinazoline,
9-bromo-3-methoxy-2-n-propyl-8H-quinazolino[4,3-b]quinazoline,
9-bromo-8H-quinazolino[4,3-b]quinazoline-2- formic acid ethyl ester,
(E)-(9-bromo-8H-quinazolino[4,3-b]quinazoline-2-yl)-4(dimethylamino)-2-butene amide hydrochloride,
(E)-(9-bromo-8H-quinazolino[4,3-b]quinazoline-2-yl)-4(dimethylamino)-2-butene amide,
9-bromo-8H-quinazolino[4,3-b]quinazoline-2-formic acid,
4-(2,3-dimethoxy-8H-quinazolina[4,3-b]quinazoline-9-yl)morpholine, and
11-chloro-2,3-dimethoxy-8-trifluoromethyl-8H-quinazolino[4,3-b]quinazoline.

29. A method of preparing an active pharmaceutical ingredient according to any one of claims 1 to 28, which comprises the steps as follow:
(I)the reagents denoted as formula I and formula II are dissolved in solvent A to form a solution, 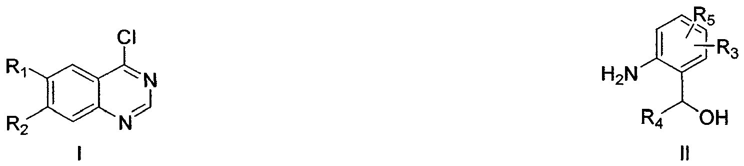
(II)the reagents base A or acid B is then added to the solution formed in step I, obtain the compound denoted as formula III from the reaction mixture, 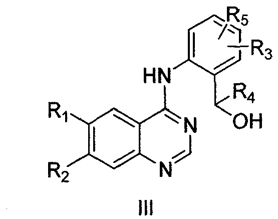
(III) the compound denoted as formula V is obtained by reacting the formula III with POCl₃ or methanesulfonyl chloride in solvent B, 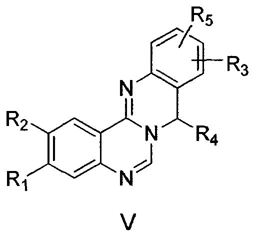 wherein, R₁, R₂ are independently chosen from hydrogen, halogen, amino, nitro, hydroxamino, carboxyl, C₁-C₄ alkoxy carbonyl, C₁-C₄ alkyl, C₁-C₄ acylamino, (E)-4-(dimethylamino)-2-butene acylamino, alkoxy, guanidino, ureido, trifluoromethyl, C₁-C₄ sulfonyl, aryl sulfonyl, substituted and unsubstituted phenyl, substituted and unsubstituted heterocyclic,
R₃, R₅ are independently chosen from hydrogen, halogen, trifluoromethyl, cyano, C₂-C₄ alkynyl, nitro, amino, hydroxyl, methylol, alkyl sulfonic ester, C₁-C₄ alkyl, C₁-C₄ alkoxy, C₁-C₄ acylamino , benzamido, C₁-C₄ substituted amino, C₁-C₄ unsaturated aliphatic chain, substituted and unsubstituted five-membered aliphatic ring, substituted and unsubstituted six-membered aliphatic ring, substituted and unsubstituted phenyl, substituted heterocyclic, substituted benzyloxy,
R₄ is chosen from hydrogen, trifluoromethyl, C₁-C₄ alkyl and C₁-C₄ alkoxy, R₁, R₂, R₃, R₄ and R₅ are not simultaneously hydrogen.

30. The method according to claim 29, wherein the solvent A is chosen from acetonitrile, isopropyl alcohol, dichloromethane, N,N-dimethylformamide, N,N-dimethylacetamide and 1,4-dioxane; the solvent B is chosen from acetonitrile, dichloromethane, chloroform, toluene and benzene; the base A is chosen from triethylamine, pyridine, potassium carbonate, and diisopropyl ethylamine; the acid B is chosen from formic acid, acetic acid, hydrochloric acid, sulfuric acid and phosphoric acid

31. The method according to claim 29, wherein the compound V is further dissolved in a hot solvent of acid/ethanol, the solution is then cooled to obtain the precipitate of the salts of compound V.

32. The method according to claim 29, wherein the acid is chosen from hydrochloric acid, hydrobromic acid, sulfuric acid, acetic acid, lactic acid, tartaric acid, tannic acid, citric acid, trifluoroacetic acid, malic acid, maleic acid, succinic acid, p-toluenesulfonic acid and methanesulfonic acid.

33. A pharmaceutical composition comprising an active pharmaceutical ingredient, or a pharmaceutically acceptable salt thereof, or a hydrate of the pharmaceutically acceptable salt according to any of claims 1 to 28, and at least one pharmaceutical accepted carrier.

34. An active pharmaceutical ingredient, a pharmaceutically acceptable salt thereof, or a hydrate of the pharmaceutically acceptable salt according to any of claims 1 to 28 for use in the treatment of cancer or the diseases caused by abnormal cell differentiation or proliferation.
